# FASCICULE DE BREVET EUROPEEN

(11) **EP 1 040 121 B1**
(45) Date de publication et mention de la délivrance du brevet: **21.07.2004**
(21) Numéro de dépôt: 98962475.4
(22) Date de dépôt: 16.12.1998
(51) Int. Cl.: C07K 7/64

(54) **NOUVEAU PROCEDE DE PREPARATION DE DERIVES DE CYCLOSPORINE**
NEUES VERFAHREN ZUR HERSTELLUNG VON CYKLOSPORINDERIVATEN
NOVEL METHOD FOR PREPARING CYLOSPORIN DERIVATIVES

(30) Priorité: 19.12.1997 FR 9716189
(43) Date de publication de la demande: 04.10.2000
(73) Titulaire: Aventis Pharma, 92160 Antony (FR)
(72) Inventeur: AMOURET, Guy, F-94260 Fresnes (FR); GUERREIRO, Antonio, F-77380 Combs la Ville (FR); VISKOV, Christian, F-91130 Ris Orangis (FR); MIGNANI, Serge, F-92290 Chatenay Malabry (FR); EVERS, Michel, F-94510 La Queue en Brie (FR); BARRIERE, Jean-Claude, F-91400 Bures sur Yvette (FR); BASHIARDES, Georges, F-94320 Thiais (FR); CARRY, Jean-Christophe, F-92190 Meudon (FR); FILOCHE, Bruno, F-94000 Créteil (FR)
(86) Numéro de dépôt international: PCT/FR1998/002745
(87) Numéro de publication internationale: WO 1999/032512

(56) Documents cités:
- EP-A- 0 194 972
- WO-A-97/04005
- D SEEBACH ET AL.: "Modification of cyclosporin A (CS). generation of an enolate at the sarcosine residue and reactions with electrophiles" JOURNAL OF THE AMERICAN CHEMICAL SOCIETY., vol. 76, 1993, pages 1564-1590, XP002022424 DC US

## Description

La présente invention se rapporte à un nouveau procédé de préparation de dérivés de cyclosporine modifiés en position -3, qui consiste à traiter une cyclosporine avec un amidure alcalin dans l'ammoniac liquide ou bien dans une amine aliphatique.

Les cyclosporines constituent un groupe de undécapeptides poly-N-méthylés cycliques qui possèdent communément des propriétés immunosuppressives, anti-inflammatoires, et anti-parasitaires.

Une des premières cyclosporines naturelles à avoir été isolée est connue sous le nom de cyclosporine A dont la structure est la suivante :

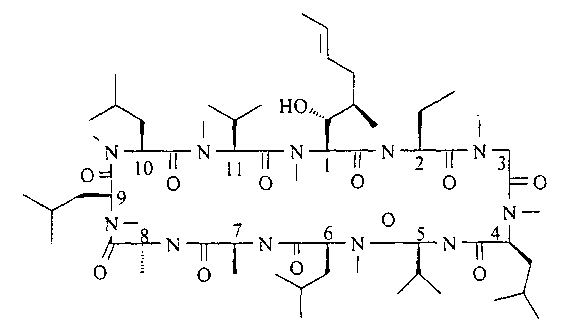

Aujourd'hui, de nombreuses cyclosporines naturelles sont connues et isolées (par exemple les cyclosporines A à Z ci-après dénommées "cyclosporines"), et de nombreuses cyclosporines de synthèse ont été préparées (ci-après dénommées "dérivés de cyclosporine").

Actuellement, les dérivés de cyclosporine modifiés en positions -3 sont obtenus selon la méthode décrite dans le brevet EP 194 972. Cette méthode consiste notamment à traiter dans un premier temps une cyclosporine avec un dérivé organométallique sous atmosphère inerte, puis à préparer dans un second temps des dérivés de cyclosporine modifiés en position -3 par addition d'un agent électrophile. Le principal inconvénient de cette méthode réside dans le fait que le rendement global est généralement extrêmement faible. Par ailleurs, il est nécessaire de travailler en présence d'un très. large excès d'électrophile. Par cette méthode, il se forme de plus l'épimère S en quantité importante par rapport à l'épimère R.

Aujourd'hui, la demanderesse a montré que le procédé selon l'invention permet d'aboutir à des dérivés de cyclosporine modifiés en position -3 avec un rendement nettement amélioré. Un autre avantage de ce procédé réside dans le fait que la réaction est menée à une température beaucoup moins basse et avec un excès d'électrophile nettement moins important, ce qui facilite la mise en oeuvre à l'échelle industrielle. La demanderesse a également mis en évidence que l'épimère R requis est formé de façon beaucoup plus sélective.

La présente invention se rapporte ainsi à un procédé de préparation d'un polyanion utile pour la préparation de dérivés de cyclosporine modifiés en position -3 caractérisé en ce que l'on traite une cyclosporine par un amidure alcalin dans un mélange binaire comprenant de l'ammoniac liquide ou une amine aliphatique de bas poids moléculaire et un cosolvant, éventuellement en présence de diméthylpropylènurée (DMPU).

Le polyanion obtenu a pour formule générale : 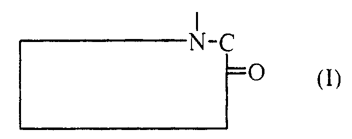 dans laquelle 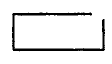 représente une cyclosporine pour laquelle un ou des groupements hydroxy libres et/ou un ou des atomes d'azote non-méthylés en position α et/ou tout autre groupement acide déprotonable sont éventuellement déprotonés, ou sont sous forme protégée.

Au sens de la présente invention, on entend par amine aliphatique de bas poids moléculaire toute amine aliphatique mono- di- ou tri-alkylée, les radicaux alkyle pouvant éventuellement former un hétérocycle saturé contenant 5 ou 6 chaînons avec l'atome d'azote auquel ils sont rattachés, ou les radicaux alkyle étant droits ou ramifiés et contenant 1 à 4 atomes de carbone. De façon avantageuse, l'amine aliphatique de bas poids moléculaire est la méthylamine.

Selon l'invention, on opère en présence de 10 à 20 moles d'amidure alcalin par mole de cyclosporine, et avantageusement entre 14 et 17 moles d'amidure alcalin par mole de cyclosporine. Notamment, l'amidure alcalin peut être l'amidure de sodium, l'amidure de potassium, le sel de lithium de l'hexaméthyldisilazane, ou le sel de sodium de l'hexaméthyldisilazane.

Généralement, on effectue la réaction dans un mélange binaire comprenant 25 % à 75 % en volume d'ammoniac liquide ou d'une amine aliphatique de bas poids moléculaire dans un cosolvant. Notamment, le cosolvant est un éther aliphatique ou cyclique, un hydrocarbure aromatique, ou la pyridine. A titre d'exemple, on peut citer le tétrahydrofuranne (THF), le *t*-butylméthyléther (TBME), le diméthoxyéthane (DME), l'anisole, le dioxanne, ou le toluène. De façon préférée, on opère dans un mélange binaire comprenant 40 % à 60 % en volume d'ammoniac liquide ou d'amine aliphatique de bas poids moléculaire, et plus préférentiellement entre 50 % et 60 % en volume d'ammoniac liquide, et encore plus préférentiellement on opère dans un mélange binaire comprenant environ 55 % en volume d'ammoniac liquide.

Le traitement de la cyclosporine par un amidure alcalin en solution dans un mélange binaire comprenat de l'ammoniac liquide ou une amine aliphatique de bas poids moléculaire et un cosolvant, éventuellement en présence de DMPU, est mis en oeuvre à une température comprise entre -38°C et la température d'ébullition de l'ammoniac liquide ou de l'amine aliphatique de bas poids moléculaire, lorsque celle-ci est inférieure à 0°C, ou bien dans le cas contraire à une température comprise entre -38°C et 0°C.

Notamment, lorsque ledit traitement est effectué dans un mélange binaire comprenant de l'ammoniac liquide, la température de la réaction est comprise entre -38°C et -32°C, et préférentiellement entre -35°C et -32°C. Encore plus préférentiellement, la réaction est mise en oeuvre à environ -33°C. Lorsque ledit traitement est effectué dans un mélange binaire comprenant de la méthylamine, la température de réaction est comprise entre -8°C et -4°C, et de façon préférée, la température est environ -6°C. Par ailleurs, le ratio (poids/poids) de cyclosporine mis en jeu par rapport au poids total de la solution contenant la cyclosporine, l'amidure alcalin, le mélange binaire comprenant de l'ammoniac liquide ou une amine aliphatique de bas poids moléculaire et un cosolvant, et éventuellement la DMPU, est généralement inférieur ou égal à 15 %. Lorsque la réaction est effectuée sans DMPU, le ratio de cyclosporine par rapport au poids total de la solution est de préférence inférieur ou égal à 6 %, et encore plus avantageusement, ce ratio est compris entre 2 % et 5 %. Lorsque la réaction est effectuée en présence de DMPU le ratio de cyclosporine par rapport au poids total de la solution est de préférence inférieur ou égal à 12 %.

Selon une variante de l'invention, on ajoute le mélange binaire d'ammoniac liquide ou d'amine aliphatique de bas poids moléculaire et de cosolvant, à un mélange contenant l'amidure alcalin sous forme solide, la cyclosporine et éventuellement la DMPU. Selon une autre alternative, on peut ajouter le mélange binaire contenant l'ammoniac liquide ou à l'amine aliphatique de bas poids moléculaire et le cosolvant à un mélange contenant la cyclosporine, éventuellement la DMPU, et le métal alcalin correspondant à l'amidure alcalin en présence d'un sel ferrique (par exemple le nitrate ferrique).

Selon un aspect préféré de la présente invention, le polyanion obtenu a pour formule : 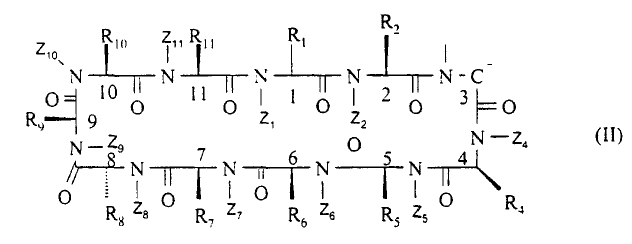 pour laquelle :
i) Les substituants R₁ à R₁₁ et Z₁ à Z₁₁ sont définis comme pour la cyclosporine A, ou bien
ii) Les substituants R₁ à R₁₁ et Z₁ à Z₁₁ sont définis comme pour la cyclosporine A. à l'exception de R₄ et Z₄ qui sont définis de façon à avoir en position -4 l'acide aminé 4'-(hydroxy)méthylleucyne (4'-(hydroxy)MeLeu), ou bien
iii) Les substituants R₂, R₅ à R₁₁ et Z₂, Z₅ à Z₁₁ sont définis comme pour la cyclosporine A, et
   - Z₁ est un groupement méthyle, et R₁ a pour formule : 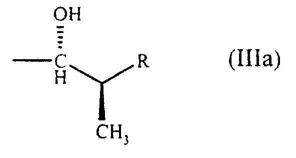
      - R étant un groupement de formule -CH₂-CH=CH-CH₂-R' pour lequel R' représente un radical alkylthio, aminoalkylthio, alkylaminoalkylthio, dialkylaminoalkylthio, pyrimidinylthio, thiazolylthio, N-alkylimidazolylthio, hydroxyalkylphénylthio, hydroxyalkylphényloxy, nitrophénylamino, ou 2-oxopyrimidin-1-yle, ou
      - R étant un groupement de formule -CH₂-S-Alk pour lequel Alk représente un groupement alkyle, et
   - Z₄ et R₄ sont des radicaux tels que l'on a en position -4 un acide aminé MeLeu ou 4'-hydroxyMeLeu, ou bien,
iv) Z₁ et R₁ sont des groupements tels que l'on a en position -1 une homothréonine substituée de formule générale :

   Rᵢ-CH₂CH(CH₃)-CH(OH)-CH(NHCH₃)-COOH (IIIb)

   dans laquelle Rᵢ représente le *n*-propyle ou le propényle et la double liaison présente de préférence une configuration trans, et
   - R₂ et Z₂ sont des radicaux tels que l'on a en position -2 l'acide alpha-aminobutyrique (αAbu), la valine (Val), la norvaline (Nva), ou la thréonine (Thr), et
   - R₄ et Z₄ sont des radicaux tels que l'on a en position -4 la N-méthyl-gamma-hydroxy-leucine ou la N-méthyl-gamma-acétyloxyleucine, et
   - R₅ et Z₅ sont des radicaux tels que l'on a en position -5 la valine, et
   - R₆, Z₆, R₉, Z₉, R₁₀ et Z₁₀ sont des radicaux tels que l'on a en position -6. -9, et -10 la N-méthylleucine, et
   - Z₇ et R₇ sont des radicaux tels que l'on a en position -7 l'alanine (Ala), et
   - Z₈ et R₈ sont des radicaux tels que l'on a en position -8 la (D)-alanine ou la (D)-sérine, et
   - Z₁₁ et R₁₁ sont des radicaux tels que l'on a en position -11 la N-méthyl-valine, ou bien,
v) Z₁ et R₁ sont des groupements tels que l'on a en position -1 un substituant méthyl-(4R)-4-[(E)-2-butènyl-4-méthyl-L-thréonine (MeBmt) ou un substituant ayant pour formule générale : 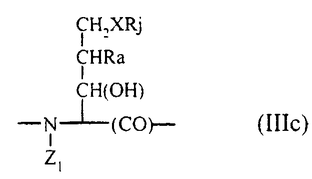 Rⱼ représentant un atome d'hydrogène, ou un groupement alkyle inférieur, un alcényle inférieur, un halogénoalkyle inférieur, un aryle, un alkyloxy inférieur, un alkoxy C₁₋₆alkyle, un hydroxyméthyle, un alkylthio inférieur, un alkylthioC ₁₋₆alkyle, un mercaptoC₁₋₆alkyle, ou un hétéroaryle, les groupements aryle et hétéroaryle pouvant être substitués avec un ou plusieurs groupements fonctionnels alkyle C₁₋₆, alcanoyle C₁₋₆, halogénoalkyle C₁₋₆, halogène, cyano, hydroxyalkyle C₁₋₃, alkyloxy C₁₋₆, alkyl-S(O)ₙ C₁₋₆ avec n = 0, 1, ou 2, NR_{b}COR_{c} avec R_{b} et R_{c} représentant indépendamment H ou un alkyle C₁₋₆, -NO₂, -NR_{b}R_{c}, -OR_{b}, -CONR_{b}R_{c}, -COR_{b}, -NR_{b}CONR_{b}R_{c}, NR_{b}COR_{c}, -OCOR_{b}, -SCOR_{b}, ou -OCH₂O-, et Rₐ étant un alkyle inférieur, Z₁ étant un alkyle inférieur, un phénylalkyle inférieur, ou un aryle, et X représentant S, SO, SO₂, 0, ou bien NR_{b}, et
   - Z₂ et R₂ sont des substituants tels que l'on a en position -2 l'acide aminé L-2-aminobutyryle, norvalyle, L-thréonyle, ou bien le même acide aminé qu'en position -1, et
   - Z₄ et R₄ sont des substituants tels que l'on a en position -4 l'acide aminé N-méthyl-L-leucyle, et
   - Z₅ et R₅ sont des substituants tels que l'on a en position -5 l'acide aminé L-valyle,
   ou norvalyle, et
   - Z₆ et R₆ sont des substituants tels que l'on a en position -6 l'acide aminé N-méthyl-L-leucyle, et
   - Z₇ et R₇ sont des substituants tels que l'on a en position -7 l'acide aminé L-alanyle, L-2-aminobutyryle, ou L-phénylalanyle, et
   - Z₈ et R₈ sont des substituants tels que l'on a en position -8 l'acide aminé D-alanyle
   ou L-alanyle, et
   - Z₉ et R₉ sont des substituants tels que l'on a en position -9 l'acide aminé N-méthyl-L-leucyle ou N-méthyl-L-valyle, et
   - Z₁₀ et R₁₀ sont des substituants tels que l'on a en position -10 l'acide aminé N-méthyl-L-leucyle ou L-leucyle, et
   - Z₁₁ et R₁₁ sont des substituants tels que l'on a en position -11 l'acide aminé N-méthyl-L-valyle, L-valyle, ou L-2-aminobutyryle, ou bien
vi) Les substituants R₄ à R₁₁, et Z₄ à Z₁₁ sont définis comme pour la cyclosporine A, et :
   - Z₁ et R₁ sont des substituants tels que l'on a en position -1 l'acide aminé MeBmt ou dihydro-MeBmt, et
   - Z₂ et R₂ sont des substituants tels que l'on a en position -2 l'acide aminé αAbu, Thr, Val, ou Nva, ou bien
vii) Les substituants R₇ à R₁₁, et Z₇ à Z₁₁ sont définis comme pour la cyclosporine A, et :
   - Z₁ et R₁ sont des substituants tels que l'on a en position -1 l'acide aminé MeBmt. dihydro-MeBmt, ou 8'-hydroxy-MeBmt, et
   - Z₂ et R₂ sont des substituants tels que l'on a en position -2 l'acide aminé αAbu, Val. Thr, Nva, ou méthyl-O-thréonine (MeOThr), et
   - Z₄ et R₄ sont des substituants tels que l'on a en position -4 l'acide aminé MeLeu, γ-hydroxy-MeLeu, Melle, MeVal, MeThr, MeAla, Mealle, ou MeaThr, et
   - Z₅ et R₅ sont des substituants tels que l'on a en position -5 l'acide aminé Val, Leu, MeVal, ou MeLeu, et
   - Z₆ et R₆ sont des substituants tels que l'on a en position -6 l'acide aminé MeLeu, γ-hydroxy-MeLeu, ou MeAla,
   à condition que lorsque R₄ et Z₄ signifient MeLeu, R₅ et Z₅ signifient alors MeVal ou MeLeu ou bien R₁ et Z₁ signifient 8'-hydroxy-MeBmt, ou bien
viii) Les substituants R₁ à R₁₁ et Z₁ à Z₁₁ définissent une cyclosporine pour laquelle le carbone en 3' du résidu en position -1 ou le carbone en β du résidu en position -2 est substitué par O-acyle ou oxo, et notamment
   - Z₁ et R₁ sont des substituants tels que l'on a en position -1 un résidu de formule générale 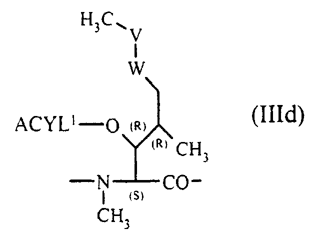 pour laquelle -v-w- est CH₂-CH₂ ou CH=CH trans et ACYL¹ représente un groupe acyle, et
   - Z₂ et R₂ sont des substituants tels que l'on a en position -2 un acide aminé αAbu, Val, Thr, Nva, ou le résidu d'un α-acide aminé β-O-acylé, et
   - Z₅ et R₅ sont des substituants tels que l'on a en position -5 un acide aminé Val, ou Nva lorsque l'on a simultanément un acide aminé Nva en position -2, et
   - Z₈ et R₈ sont des substituants tels que l'on a en position -8 un acide aminé (D)-Ala, un résidu d'un α-acide aminé β-O-acylé ou β-hydroxylé ayant la configuration (D). et
   - les substituants en position -4, -6, -7, et -9 à -11 sont définis comme pour la cyclosporine A,
   un ou des groupements hydroxy et/ou un ou des atomes d'azote non-méthylés en position α et/ou tout autre groupement acide déprotonable présents dans ladite formule générale (II) étant éventuellement déprotonés, ou étant sous forme protégée.

Les polyanions résultants du procédé selon l'invention sont particulièrement intéressants comme intermédiaires pour la préparation de dérivés de cyclosporine de formule générale : 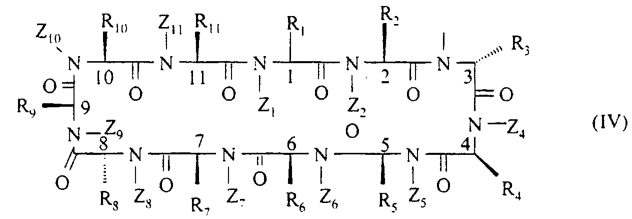 pour laquelle :
1) les substituants R₁ à R₁₁ et Z ₁ à Z₁₁ sont tels que définis ci-avant en i), et R₃ représente un substituant -S-Alk-R^{o} pour lequel :
   - Alk représente un radical alkylène contenant 2 à 6 atomes de carbone en chaîne droite ou ramifiée ou cycloalkylène contenant 3 à 6 atomes de carbone et
   - R° représente
      - soit un radical carboxy ou alkyloxycarbonyle,
      - soit un radical -NG₁G₂ pour lequel G₁ et G₂, identiques ou différents, représentent des atomes d'hydrogène, ou des radicaux alkyle, alcényle (2 à 4C), cycloalkyle (3 à 6C), phényle éventuellement substitué (par un atome d'halogène, alkyloxy, alkyloxycarbonyle, amino. alkylamino ou dialkylamino), ou représentent des radicaux benzyle ou hétérocyclyle saturé ou insaturé contenant 5 ou 6 chaînons et 1 à 3 hétéroatomes, ou pour lequel G₁ et G₂ forment avec l'atome d'azote auquel ils sont rattachés un hétérocycle contenant 4 à 6 chaînons, saturé ou insaturé, pouvant contenir un autre hétéroatome choisi parmi l'azote, l'oxygène ou le soufre et éventuellement substitué par alkyle, phényle ou benzyle,
      - soit un radical de formule générale : 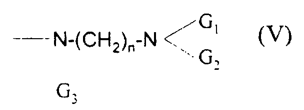 pour lequel G₁ et G₂ sont définis comme ci-dessus, G₃ représente un atome d'hydrogène ou un radical alkyle et n est un nombre entier de 2 à 4, les portions ou radicaux alkyle définis ci-dessus étant droits ou ramifiés et contenant 1 à 4 atomes de carbone. ou bien
2) les substituants R₁ à R₁₁ et Z₁ à Z₁₁ sont tels que définis ci-avant en ii), et R₃ représente -S-CH₃ ou un substituant -S-Alk-R° pour lequel :
   - Alk représente un radical alkylène contenant 2 à 6 atomes de carbone en chaîne droite ou ramifiée ou cycloalkylène contenant 3 à 6 atomes de carbone et
   - R° représente
      - soit un radical hydroxy, carboxy ou alkyloxycarbonyle,
      - soit un radical -NG₁G₂ ou un radical de formule générale : 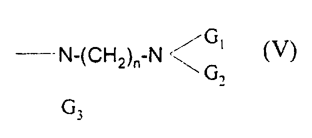 tels que définis ci-avant,
3) les substituants R₁ à R₁₁ et Z₁ à Z₁₁ sont tels que définis ci-avant en iii), et R₃ est un radical de structure -S-Alk-R° pour lequel :
   - Alk représente un radical alkylène contenant 2 à 6 atomes de carbone en chaîne droite ou ramifiée ou cycloalkylène contenant 3 à 6 atomes de carbone et
   - R° représente
      - soit un atome d'hydrogène, un radical hydroxy, carboxy ou alkyloxycarbonyle,
      - soit un radical -NG₁G₂ pour lequel G₁et G₂ identiques ou différents représentent des atomes d'hydrogène, ou des radicaux alkyle, cycloalkyle (3 à 6C), phényle éventuellement substitué (par un atome d'halogène, alkyloxy, alkyloxycarbonyle. amino, alkylamino ou dialkylamino), ou représentent des radicaux benzyle ou hétérocyclyle saturé ou insaturé contenant 5 ou 6 chaînons et 1 à 3 hétéroatomes, ou pour lequel G₁ et G₂ forment avec l'atome d'azote auquel ils sont rattachés un hétérocycle à 5 ou 6 chaînons pouvant contenir un autre hétéroatome choisi parmi l'azote, l'oxygène ou le soufre et éventuellement substitué par alkyle,
      - soit un radical de formule générale : 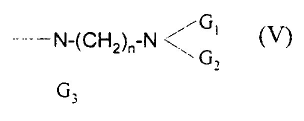 tel que défini précédemment, ou bien
4) les substituants R₁ à R₁₁ et Z 1 à Z₁₁ sont tels que définis ci-avant en iv), et R₃ est un substituant :
   - alkyle en C₂ à C₆, alcényle, alcynyle, linéaires ou ramifiés, ces groupes pouvant encore être substitués par un groupe hydroxy, amino, alkylamino en C₁ à C₄, dialkylamino en C₁ à C₃, alkyloxy, ou acyloxy, ou
   - COOG₄ ou CONHG₄, G₄ étant un alkyle contenant 1 à 4 atomes de carbone linéaire ou ramifié, ou
   - -Y-G₅ dans laquelle Y représente un atome de soufre S ou d'oxygène O, et G₅ est un alkyle en C₁ à C₄, un alcényle ou un alcynyle, linéaires ou ramifiés, et dans laquelle si Y est un atome de soufre S, G₅ peut être aussi un aryle ou un hétéroaryle, ou
   - un groupement halogène, ou cyano, ou
   - CHG₆G₇, dans laquelle G₆ est un atome d'hydrogène, un groupement méthyle, éthyle, phényle, et G₇ est un atome d'hydrogène, ou un groupe hydroxy, halogène, amino, alkylamino en C₁ à C₄, dialkylamino en C₁ à C₄, acyloxy, *t*-butoxycarbonylamino-éthoxy-éthoxy-acétyloxy, ou alkyloxycarbonyle, ou bien
5) Les substituants R₁ à R₁₁ et Z₁ à Z₁₁ sont tels que définis ci-avant en v), et R₃ est un groupement tel que l'on a en position -3 un résidu α-(méthylmercapto)-sarcosyl ou N-méthyl-(D)-alanyl, ou bien
6) Les substituants R₁ à R₁₁ et Z₁ à Z₁₁ sont tels que définis ci-avant en vi). et R₃ est un groupement C ₁₋₆alkyle, halogénoC₁₋₆alkyle, hydroxyC₁₋₆alkyle, mercaptoC₁₋₆alkyle, amino C₁₋₆alkyle, C₂₋₅alkoxycarbonyl-amino-(C₁₋₄alkyle), nitroC₁₋₆alkyle, cyanoC₁₋₅alkyle, C₁₋₆alkoxy-(C₁₋₆alkyle), C₁₋₆alkylthio-(C₁₋₆ alkyle), C₂₋₇alcanoyloxy-(C₁₋₆alkyle), C₂₋₇diazoalcanoyloxy-(C₁₋₆alkyle), carboxy-(C₁₋₆alkyle), C₂₋₇alkoxycarbonyl-(C₁₋₆alkyle), aminocarbonyl-(C₁₋₄ alkyle), aminocarbonyloxy-(C₁₋₄alkyle), amino-(C₁₋₄alcanoyloxy)-(C₁₋₄al-kyle). amino-(C₂₋₉alkoxycarbonyl)-(C₁₋₄alkyle), C₂₋₇alkylcarbonyle, C₂₋₇ alkoxycarbonyle, C ₁₋₆alkylthio, hydroxy-C₁₋₆(alkylthio, C ₁₋₆alkoxy-(C₁₋₆ alkylthio), C₂₋₁₁alcanoyloxy-(C₂₋₄alkylthio), C₂₋₁₁alcanoyloxy-(C₂₋₄ alkylsulfinyle), C₂₋₁₁-alcanoyloxy-(C₂₋₄alkylsulfonyle), aminocarbonyloxy-(C₂₋₄ alkylthio), C₂₋₁₁amino-alcanoyloxy-(C₂₋₄alkylthio), aminocarbonyloxy-(C₂₋₄ alkylsulfinyle), aminocarbonyl-oxy-(C₂₋₄alkylsulfonyle), aminoalcanoyloxy-(C₂₋₄ alkylsulfinyle), aminoalcanoyloxy-(C₂₋₄alkylsulfonyle), aminocarbonyle, C₃₋₆ alcényle, C₃₋₆alcynyle, halogénoC₃₋₆alcényle, halogénoC₃₋₆alcynyle, hydroxyC₃₋₆ alcényle, aryl-(C₁₋₆alkyle), aryl-(C₁₋₆alkyle) hydroxylé, aryl-(C₃₋₆alcényle), aryl-(C₃₋₆alcynyle), aryl-(C₃₋₆alcényle) hydroxylé, aryl-(C₃₋₆alcynyle) hydroxylé, arylthio, hétéroarylthio, aryl-(C₂₋₅alkoxycarbonylamino)-( C₁₋₄alkyle), halogène, cyano, ou un groupe de formule Q-(CH₂-CH₂-O)ₙ-CO-O-CH₂- pour laquelle n est 1, 2, ou 3, et Q est amino, ou bien
7) les substituants R₁ à R₁₁ et Z₁ à Z₁₁ sont tels que définis ci-avant en vii), et R₃ est un groupement tel que l'on a en position -3 un acide aminé (D)-MeAla, ou bien
8) les substituants R₁ à R₁₁ et Z₁ à Z₁₁ sont tels que définis ci-avant en viii), et R₃ est un groupement tel que l'on a en position -3 un α-acide aminé N-méthylé en position α et ayant la configuration (D).

Les dérivés de cyclosporine de formule générale (IV) peuvent être obtenus par addition d'un agent électrophile sur le polyanion. Lorsque les substituants de cet agent peuvent interférer avec la réaction, il est préférable de les protéger préalablement avec des radicaux compatibles et pouvant être mis en place et éliminés sans toucher au reste de la molécule. L'addition peut être suivie le cas échéant des étapes de séparation et de purification du dérivé de cyclosporine de formule (IV) selon les méthodes connues de l'homme du métier.

Ainsi, un autre objet de la présente invention se rapporte à un procédé de préparation de dérivés de cyclosporine substitués en position -3 caractérisé en ce que l'on prépare un polyanion par traitement par un amidure alcalin d'une cyclosporine dans un mélange binaire comprenant de l'ammoniac liquide ou une amine aliphatique de bas poids moléculaire et un cosolvant, éventuellement en présence de diméthylpropylènurée, puis on additionne un agent électrophile, les radicaux hydroxy présents sur la cyclosporine et/ou les substituants de l'agent électrophile pouvant éventuellement interférer avec la réaction étant préalablement protégés, et élimine le cas échéant les radicaux protecteurs et/ou transforme éventuellement le produit obtenu en un sel lorsqu'ils existent.

Les dérivés de cyclosporine de formule générale (IV) dont les substituants sont définis comme en 1), 2), 3), ou 4) pour lesquels R₃ est S-G₅ peuvent être obtenus par addition sur un polyanion de formule générale (II) d'un disulfure de formule générale :

G-S-S-G (VI)

pour lequel G est Alk-R° ou G₅ tels que définis ci-avant en 1), 2), 3), ou 4), les fonctions du polyanion pouvant interférer avec la réaction ayant été le cas échéant préalablement protégées, suivie de l'élimination le cas échéant du/des radicaux protecteurs.

Le disulfure de formule générale (VI) est généralement additionné soit pur soit en solution dans un solvant organique tel qu'un hydrocarbure (toluène par exemple) ou un éther aliphatique ou cyclique (par exemple le dioxanne. le diéthyléther, le tétrahydrofuranne ou le *t*-butylméthyléther), à une température comprise entre -38°C et la température d'ébullition de l'ammoniac liquide ou de l'amine aliphatique de bas poids moléculaire, et en tout état de cause à température inférieure à 0°C. Notamment on opère à une température comprise entre -35°C et -32°C, et préférentiellement à - 33°C, lorsque le solvant dans lequel le polyanion est préparé est l'ammoniac liquide. Si le solvant utilisé pour la préparation du polyanion est la méthylamine, on opère à une température comprise entre -8°C et -4°C, et de préférence à -6°C.

Le disulfure de formule générale (VI) peut être obtenu à partir de deux molécules d'un composé de formule générale G-SH pour lequel G est Alk-R° ou G₅ tels que définis précédemment en 1), 2), 3), ou 4). On opère en milieu oxydant, dans un solvant organique (par exemple dans l'oxyde de diéthyle ou dans le dichlorométhane) ou dans un alcool (par exemple le méthanol ou l'éthanol), et en présence d'un hydroxyde alcalin. Notamment, le milieu oxydant est obtenu par passage d'oxygène ou par adjonction de diiode dans un solvant organique, par exemple l'oxyde de diéthyle. De façon préférée, l'hydroxyde alcalin est l'hydroxyde de sodium.

Lorsque les substituants du radical G peuvent interférer avec la réaction, il est préférable de les protéger préalablement par des radicaux compatibles et pouvant être mis en place et éliminés sans toucher au reste de la molécule. De plus les radicaux hydroxy présents sur la cyclosporine peuvent être éventuellement protégés par tout groupement qui n'interfère pas avec la réaction.

A titre d'exemple les groupements protecteurs peuvent être choisis parmi les radicaux décrits par T.W. GREENE, Protective Groups in Organic Synthesis, J. Wiley-Interscience Publication (1991) ou par Mc Omie, Protective Groups in Organic Chemistry, Plenum Press (1973).

Dans la formule générale (V), lorsque G₁ et/ou G₂ représentent un substituant hétérocyclyle, celui-ci peut être avantageusement choisi parmi pyridyle, tétrahydropyridyle, pipéridyle, imidazolyle, oxazolyle. thiazolyle.

Lorsque G ₁ et G₂ forment un hétérocycle avec l'atome d'azote auquel ils sont rattachés, le radical hétérocyclyle peut être choisi à titre d'exemple parmi azétidinyle, pipéridyle, pipérazmyle, N-méthyl pipérazinyle, N-phényl pipérazinyle, N-benzyl pipérazinyle, pyridyle, imidazolyle, morpholino, thiomorpholino, tétrahydropyridyle, méthyl tétrahydropyridyle (par exemple 4-méthyl tétrahydropyridyle), phényl tétrahydropyridyle (par exemple 4-phényl tétrahydropyridyle).

La réaction de thioalkylation peut être suivie le cas échéant des étapes de séparation et de purification du dérivé de cyclosporine de formule générale (IV) selon les méthodes connues de l'homme du métier. Notamment, on peut opérer par des méthodes telles que la cristallisation ou la chromatographie.

Les dérivés de formule générale (IV) pour laquelle les substituants R₁ à R₁₁ et Z₁ à Z₁₁ sont tels que définis en 4) à l'exception de R₃ représentant S-G₅, peuvent être obtenus à partir du polyanion selon l'invention par les méthodes décrites dans la demande de brevet WO 97/04005 ou par toute autre méthode équivalente.

Les dérivés de formule générale (IV) pour laquelle les substituants R₁ à R₁₁ et Z₁ à Z₁₁ sont tels que définis en 5) peuvent être obtenus à partir du polyanion selon l'invention par analogie avec les méthodes décrites dans la demande de brevet EP 194 972.

Les dérivés de formule générale (IV) pour laquelle les substituants R₁ à R₁₁ et Z₁ à Z₁₁ sont tels que définis en 6) peuvent être obtenus à partir du polyanion selon l'invention par les méthodes décrites dans la demande de brevet EP 194 972 ou par toute autre méthode équivalente.

Les dérivés de formule générale (IV) pour laquelle les substituants R₁ à R₁₁ et Z₁ à Z₁₁ sont tels que définis en 7) peuvent être obtenus à partir du polyanion selon l'invention par analogie avec les méthodes décrites dans la demande de brevet EP 194 972.

Les dérivés de formule générale (IV) pour laquelle les substituants R₁ à R₁₁ et Z₁ à Z₁₁ sont tels que définis en 8) peuvent être obtenus à partir du polyanion selon l'invention par analogie avec les méthodes décrites dans la demande de brevet EP 194 972.

Il est entendu que les dérivés de cyclosporine obtenus peuvent être éventuellement transformés en sels lorsqu'ils existent.

Les dérivés de cyclosporine tels que définis en 1), 2), 3), 4), et 7) sont utiles pour le traitement et/ou la prophylaxie des infections à rétrovirus, et plus particulièrement du SIDA (syndrome d'immuno-déficience acquise) et de syndromes associés [ARC (AIDS related complex)]. Ils présentent l'avantage d'être très faiblement immunosuppresseurs.

Les dérivés de cyclosporine tels que définis en 5) présentent une activité immunosuppressive, et sont donc utiles pour le traitement de diverses maladies inflammatoires chroniques et des maladies autoimmunes.

Les dérivés de cyclosporine tels que définis en 6) et en 8) possèdent :
- soit une activité immunosuppressive, et ils sont donc utiles notamment pour le traitement et/ou la prophylaxie des maladies auto-immunes ou pour prévenir le rejet d'organes transplantés,
- soit une activité anti-inflammatoire, et ils sont donc notamment utiles pour le traitement d'inflammations comme par exemple l'arthrite et les maladies rhumatismales,
- soit une activité anti-parasitaire, et ils sont par exemple utiles pour le traitement de la schistosomiasis, la filariasis, la leishmaniasis, la coccidioidomycosis. ou de la malaria.

Les exemples suivants, donnés à titre non-limitatif, illustrent la présente invention.

### Exemple 1

La [(R)-2-(hydroxy)éthylthio-Sar]³ cyclosporine A peut être préparée selon la méthode suivante :
A 120 cm³ d'ammoniac maintenus à une température voisine de -33°C, on ajoute 100 mg de sodium métallique puis 100 mg de nitrate ferrique. Dès que la coloration bleue du milieu a disparu, 1,3 g de sodium métallique sont ajoutés en 45 minutes. Le mélange est agité à -33°C pendant 30 minutes, puis une solution de 4,8 g de cyclosporine A dans 120 cm³ de tétrahydrofuranne est ajoutée goutte à goutte en approximativement 30 minutes. Le mélange est agité 30 minutes à une température voisine de -33°C puis une solution de 30,56 g de disulfure de [2-(*t*-butyldiméthylsililoxy)éthyle] dans 30 cm³ de tétrahydrofuranne est ajoutée en 20 minutes. Le mélange réactionnel est agité à une température voisine de -33°C durant 2 heures, puis 4 g de chlorure d'ammonium solide sont ajoutés par portions. Sous agitation, on laisse s'évaporer l'ammoniac en faisant passer la température du mélange de -33°C à 25°C en 19 heures. Le mélange est dilué par 120 cm³ d'eau distillée puis 120 cm³ d'éther de diéthyle. La phase organique est décantée, et la phase aqueuse est extraite par 240 cm³ au total d'éther de diéthyle. Les phases organiques réunies sont lavées par 240 cm³ au total d'eau distillée, séchées sur sulfate de magnésium, filtrées puis concentrées à sec sous pression réduite (2,7 kPa) à une température voisine de 40°C. Le solide résiduel (28,47 g) est purifié par chromatographie sur une colonne de silice (0,020-0,045 mm; éluant : acétate d'éthyle/méthanol 4:1 en volumes) et en recueillant des fractions de 100 cm³. Les fractions contenant le produit attendu (fractions 58 à 95) sont réunies et concentrées à sec sous pression réduite (2,7 kPa) à une température voisine de 40°C pour donner 1,44 d'une meringue. Une aliquote de 0,2 g de cette meringue est traitée par 2 cm³ d'éther de diéthyle durant 12 heures. Après séchage sous pression réduite (3 kPa) à une température voisine de 20°C, on obtient 0,13 g de [(R)-2-(*t*-butyldiméthylsililoxy)éthylthio-Sar]³ cyclosporine A sous la forme d'un solide beige fondant à une température voisine de 125°C qui sont engagés dans l'étape suivante. A une solution de 1,02 g de [(R)-2-(*t*-butyldiméthylsililoxy)éthylthio-Sar]³ cyclosporine A dans 50 cm³ de tétrahydrofuranne on ajoute 3,7 cm³ d'une solution 1 M de fluorure de tétrabutylammonium dans le tétrahydrofuranne. Le mélange est agité durant 4 heures à une température voisine de 20°C, puis 50 cm³ d'eau distillée sont ajoutés au mélange. Le tétrahydrofuranne est évaporé sous pression réduite (2,7 kPa) à une température voisine de 40°C. Le résidu est traité par 50 cm³ d'acétate d'éthyle. La phase organique est décantée et la phase aqueuse est extraite par 100 cm³ au total d'acétate d'éthyle. Les phase organiques réunies sont lavées par 100 cm³ au total d'eau distillée, séchées sur sulfate de magnésium, filtrées puis concentrées à sec sous pression réduite (2,7 kPa) à une température voisine de 40°C. Le solide résiduel (0,88 g) est purifié par chromatographie sur une colonne de silice (0,020-0,045 mm; éluant : acétate d'éthyle) en recueillant des fractions de 15 cm³. Les fractions contenant le produit attendu (fractions 62 à 140) sont réunies et concentrées à sec sous pression réduite (2,7 kPa) à une température voisine de 40°C. Le solide résiduel (0,82 g) est purifié par chromatographie sur une colonne d'alumine neutre (éluant : acétate d'éthyle/cyclohexane 4:1 en volumes) en recueillant des fractions de 5 cm³. Les fractions contenant le produit attendu (fractions 18 à 288) sont réunies et concentrées à sec sous pression réduite (2,7 kPa) à une température voisine de 40°C. Le solide résiduel (0,66 g) est traité par 50 cm³ d'acétate d'éthyle, la solution est filtrée puis évaporée sous pression réduite (2,7 kPa) à une température voisine de 40°C. Le solide résiduel est trituré avec 50 cm³ d'éther de diéthyle, puis filtré et séché sous pression réduite (3 kPa) à une température voisine de 45°C pour donner 0,65 g de [(R)-2-(hydroxy)éthylthio-Sar]³ cyclosporine A sous la forme d'un solide blanc fondant à une température voisine de 139°C.

Spectre de R.M.N. ¹H (400 MHz, CDCl₃, δ en ppm) : 1,26 (d, J = 7,5 Hz, 3H : CH₃ 8β) ; 1.36 (d, J = 7,5 Hz, 3H : CH₃ 7β) ; 1,64 (d, J = 5 Hz, 3H : CH₃ 1η) ; 2,70 - 3,13 - 3,14 - 3,27 - 3,47 et 3,51 (6 s, respectivement 6H - 3H - 3H - 3H - 3H - 3H : les 7 NCH₃) ; 2,81 (t, J = 6 Hz, 2H : SCH₂) ; 3,78 (mt, 1H : CH 1β) ; 3,86 (mt, 2H : CH₂O) ; 4,54 (mt, 1H : CH 7α) ; 4,66 (t large, J = 9 Hz, 1H : CH 5α) ; 4,84 (mt, 1H : CH 8α) ; 4,99 (dd, J = 9 et 6 Hz, 1H : CH α d'une leucine) ; de 5,00 à 5,10 (mt. 2H : CH α d'une leucine et CH 2α) ; 5,13 (d, J = 11 Hz, 1H : CH 11α) ; 5,24 (dd. J = 11 et 4 Hz, 1 H : CH α d'une leucine) ; 5,33 (mt, 2H : CH=CH) ; 5,48 (d, J = 6 Hz, 1H : CH 1α) ; 5,71 (dd, J = 10,5 et 4 Hz, 1H : CH α d'une leucine) ; 5,97 (s, 1H : CH 3α) ; 7,18 (d, J = 8 Hz, 1H : CONH en 8) ; 7,30 (d, J = 9 Hz, 1H : CONH en 5) ; 7.68 (d, J = 8 Hz, 1H : CONH en 7) ; 7,98 (d, J = 10 Hz, 1H : CONH en 2).

Le disulfure de [2-(*t*-butyldiméthylsililoxy)éthyle] peut être préparé selon la méthode suivante :
A une solution de 15 cm³ de disulfure de di-2-(hydroxy)éthyle dans 20 cm³ de diméthylformamide refroidie à une température voisine de 0°C on ajoute goutte à goutte 38,64 g d'imidazole en solution dans 20 cm³ de diméthylformamide. Le mélange est agité durant 30 minutes à 0°C, puis une suspension de 93 g de *t*-butyldiméthylchlorosilane dans 200 cm³ de diméthylformamide est ajouté en maintenant la température voisine de 0°C. Le mélange est agité 30 minutes à une température voisine de 0°C puis le mélange est réchauffé à une température voisine de 25°C en 12 heures. Le mélange est concentré sous pression réduite (2,7 kPa) à une température voisine de 65°C. L'huile résiduelle est reprise par 100 cm³ de dichlorométhane et 100 cm³ d'eau distillée. La phase organique est décantée et la phase aqueuse est extraite par 300 cm³ au total de de dichlorométhane. Les phases organiques sont réunies, séchées sur sulfate de magnésium, filtrées puis concentrées sous pression réduite (2,7 kPa) à une température voisine de 45 °C. Après élimination des composés volatils par distillation sous pression réduite (20 kPa) jusqu'à une température voisine de 200°C, 41 g de disulfure de [2-(*t*-butyldiméthylsililoxy)éthyle] sont obtenus sous la forme d'une huile jaune utilisée dans l'étape précédente sans autre purification.

### Exemple 2

Le sel de méthanesulfonate de la [(R)-2-(N,N-diméthylamino)éthylthio-Sar]³ cyclosporine A peut être préparée selon la méthode suivante :
A 500 cm³ d'ammoniac maintenus à une température voisine de -33°C, on ajoute 100 mg de sodium métallique puis 100 mg de nitrate ferrique. Dès que la coloration bleue du milieu a disparu, 7,05 g de sodium métallique sont ajoutés en 45 minutes. Le mélange est agité à -33°C pendant 20 minutes, puis une solution de 25 g de cyclosporine A dans 500 cm³ de tétrahydrofuranne est ajoutée goutte à goutte en approximativement 40 minutes. Le mélange est agité 1 heure à une température voisine de -33°C puis 85 g de disulfure de [2-(N,N-diméthylamino)éthyle] sont ajoutés en 15 minutes. Le mélange réactionnel est agité à une température voisine de - 33°C durant 2 heures, puis 78 g de chlorure d'ammonium solide sont ajoutés par portions en 15 minutes. Sous agitation, on laisse évaporer l'ammoniac en faisant passer la température du milieu de -33°C à 25°C en 12 heures. Le mélange est filtré, le solide est rincé par de l'éther de diéthyle. Les phases organiques réunies sont concentrées sous pression réduite (2,7 kPa) à une température voisine de 35°C. Le résidu huileux orange obtenu (108 g) est traité par 4 litres d'eau distillée. 200 cm³ d'une solution aqueuse d'acide chlorhydrique 5N et 600 cm³ d'éther de diéthyle. La phase organique est décantée. La phase aqueuse est extraite par 1 litre au total d'éther de diéthyle puis neutralisée par ajout de bicarbonate de sodium solide jusqu'à saturation. Un litre d'éther de diéthyle est ajouté à la phase aqueuse neutralisée. La phase organique est décantée, et la phase aqueuse est extraite par 1 litre au total d'éther de diéthyle. Les phases organiques réunies sont lavées par 500 cm³ au total d'une solution saturée en chlorure de sodium, séchée sur sulfate de sodium, filtrées puis évaporées à sec sous pression réduite (2,7 kPa), à une température voisine de 35°C pour conduire à 20,9 g de [(R)-2-(N,N-diméthylamino)éthylthio-Sar]³ cyclosporine A brute sous la forme d'une meringue orange. Une aliquote de 3 g de la [(R)-2-(N,N-diméthylamino)éthylthio-Sar]³ cyclosporine A brute est purifiée par chromatographre sur une colonne d'alumine neutre (éluant : cyclohexane/acétate d'éthyle 1:4 en volume) et en recueillant des fractions de 80 cm³. Les fractions contenant le produit attendu (fractions 3 à 12) sont réunies et concentrées à sec sous pression réduite (2,7 kPa) à une température voisine de 35°C pour donner 1,78 g d'une meringue crème qui est mise en solution dans 9 cm³ d'éther de diéthyle. A cette solution sont ajoutés 6,8 cm³ d'une solution 0,2 N d'acide méthanesulfonique dans l'éther de diéthyle. Après une heure d'agitation à une température voisine de 20°C, le mélange est filtré. Le solide est rincé par 3 fois 1,8 cm³ d'éther de diéthyle. Après séchage du solide à poids constant sous pression de 5 kPa à une température voisine de 30°C durant 24 heures, on obtient 1,45 g de sel de méthanesulfonate de la [(R)-2-(N,N-diméthylamino)éthylthio-Sar]³ cyclosporine A sous la forme d'un solide beige fondant à une température voisine de 155°C.

Spectre de R.M.N. ¹H (400 MHz, (CD₃)₂SO d6, δ en ppm) : 1,21 (d, J = 7,5 Hz, 3H : CH₃ 8β) ; 1,29 (d, J = 7,5 Hz, 3H : CH₃ 7β) ; 1,69 (d, J = 6,5 Hz, 3H : CH₃ 1η) ; 1,99 (mt, 1H : CH 5β) ; 2,35 (s, 3H : CH₃ du méthanesulfonate) ; de 2,45 à 2,70 (mt, 2H : SCH₂ du 2-diméthyl aminoéthylthio en 3α) ; 2,64 - 2,80 - 2,86 - 2,93 - 2,99 et 3,17 (6 s, respectivement 3H - 6H - 9H - 3H - 3H et 3H : 7 NCH₃ et NCH₃ du 2-diméthyl aminoéthylthio en 3α) ; de 3,25 à 3,40 (mt, 2H : CH₂N du 2-diméthyl aminoéthylthio en 3α) ; 3,99 (mt, 1H : CH 1β) ; 4,15 (mt, 1H : CH 7α) ; 4,26 (t, J = 9 Hz, 1H : CH 5α) ; 4,42 (s large, 1H : OH en 1β) ; 4,79 (mt, 1H : CH 8α) ; 4,89 (mt, 1H : CH 2α) ; de 5.00 à 5,15 (mt, 1H : CH α d'une leucine) 5,11 (d, J = 11 Hz, 1H : CH 11α) ; 5,23 (mt, 2H : CH 1α et CH α d'une leucine) ; 5,33 (dd, J = 10 et 5 Hz, 1H : CH α d'une leucine) ; de 5,30 à 5,50 et 5,62 (2 mts, 1 H chacun : CH=CH) ; 5,48 (dd, J = 11 et 5 Hz, 1H : CH α d'une leucine) ; 6,87 (s, 1H : CH 3α) ; 7,64 (d, J = 7,5 Hz, 1H : CONH en 7) ; 8,24 (d, J = 9,5 Hz, 1H : CONH en 2) ; 8,28 (d, J = 8 Hz, 1H : CONH en 8) ; 8,68 (d, J = 9 Hz, 1H : CONH en 5) ; 9,28 (mf, 1H : SO₃H du méthanesulfonate)

### Exemple 3

La [(R)-2-(1-imidazolyl)éthylthio-Sar]³ cyclosporine A est préparée selon la méthode suivante :
A 80 cm³ d'ammoniac maintenus à une température voisine de -33°C, on ajoute 100 mg de sodium métallique puis 100 mg de nitrate ferrique. Dès que la coloration bleue du milieu a disparu, 0,82 g de sodium métallique sont ajoutés en 15 minutes. Le mélange est agité à -33°C pendant 15 minutes, puis une solution de 2,4 g de cyclosporine A dans 10 cm³ de tétrahydrofuranne est ajoutée goutte à goutte en approximativement 15 minutes, et 5 g de disulfure de di-[2-(1-imidazolyl)éthyle] solide sont ajoutés par portions en 15 minutes. Le mélange réactionnel est agité à une température voisine de -33°C durant 3 heures, puis 3,4 g de chlorure d'ammonium solide sont ajoutés par portions. Sous agitation, on laisse évaporer l'ammoniac en faisant passer la température du mélange de -33°C à 25°C en 12 heures. Le mélange est dilué par 100 cm³ d'eau distillée. La phase organique est décantée, et la phase aqueuse est lavée par 3 fois 50 cm³ d'acétate d'éthyle. Les phases organiques réunies sont séchées sur sulfate de magnésium, filtrées, puis concentrées à sec sous pression réduite (2,7 kPa), à une température voisine de 40°C. La meringue beige ainsi obtenue (2.25 g) est purifiée par chromatographie sur une colonne de silice (0,020-0,045 mm; éluant : acétate d'éthyle/méthanol 19:1 en volumes) et en recueillant des fractions de 20 cm³. Les fractions contenant le produit attendu sont concentrées à sec sous pression réduite (2,7 kPa) à une température voisine de 40°C pour donner 0,520 d'un solide. Ce solide, trituré avec 50 cm³ de pentane, donne, après filtration et séchage à une température voisine de 40°C, 0,420 g d'un solide qui est purifié par chromatographie sur une seconde colonne de silice (0,020-0,045 mm ; éluant : acétate d'éthyle/méthanol 4:1 en volumes). Les fractions contenant le produit attendu sont concentrées à sec sous pression réduite (2,7 kPa) à une température voisine de 40°C. Le solide résiduel est trituré avec 10 cm³ de pentane pour donner après filtration est séchage à une température voisine de 40°C 0,245 g de [(R)-2-(1-imidazolyl)-éthylthio-Sar]³ cyclosporine A sous la forme d'un solide jaune fondant vers 208°C.

Spectre de R.M.N. ¹H (400 MHz, CDCl₃, à une température de 333 K, δ en ppm) : 1.26 (d, J = 7,5 Hz, 3H : CH₃ 8β) ; 1,36 (d, J = 7,5 Hz, 3H : CH₃ 7β) ; 1,63 (d, J = 5 Hz. 3H : CH₃ 1η) ; 2,72 - 3,07 - 3,15 - 3,25 - 3,40 et 3,50 (6 s, respectivement 6H - 3H - 3H - 3H - 3H - 3H : les 7 NCH₃) ; de 3,80 à 3,95 (mt, 2H : CH 1β et OH en 1β) ; 4,16 (mt, 2H : NCH₂) ; 4,51 (mt, 1H : CH 7α) ; 4,71 (t large, J = 9 Hz, 1H : CH 5α) ; 4,86 (mt, 1H : CH 8α) ; de 4,95 à 5,10 (mt, 3H : CH α de deux leucines et CH 2α) ; 5,17 (d, J = 11 Hz, 1H : CH 11α) ; 5,22 (dd, J = 11,5 et 4 Hz, 1H : CH α d'une leucine) ; 5,35 (mt, 2H : CH=CH) ; 5,42 (d, J = 6 Hz, 1H : CH 1α) ; 5,72 (dd. J = 10.5 et 4 Hz, 1H : CH α d'une leucine) ; 5,82 (s, 1H : CH 3α) ; 6,93 - 7,10 et 7.54 (3 s larges. 1H chacun : H aromatiques de l'imidazole) ; 7,12 (d, J = 8 Hz, 1H : CONH en 8) ; 7.19 (d. J = 9 Hz, 1H : CONH en 5) ; 7,53 (mt, 1H : CONH en 7) : 7,87 (d, J = 10 Hz, 1H: CONH en 2).

Le disulfure de di-[2-(1-imidazolyl)éthyle] peut être préparé de la manière suivante :
A une solution de 15 g de 2-(1-imidazolyl)-éthanethiol dans 200 cm³ de dichlorométhane refroidie à 0°C, on ajoute goutte à goutte en 10 minutes, 32.3 cm³ de triéthylamine puis une solution de 14,59 g d'iode dans 68 cm³ d'éther de diéthyle. Le mélange est agité durant 30 minutes, à une température voisine de 20°C puis concentré à sec sous pression réduite (2,7 kPa) à une température voisine de 40°C. Le résidu pâteux obtenu est trituré en présence de 50 cm³ d'isopropanol. Le solide formé est filtré et rincé avec 25 cm³ au total d'isopropanol pour donner un premier jet de disulfure de di-[2-(1-imidazolyl)éthyle]. Les phases organiques réunies sont concentrées à sec sous pression réduite (2,7 kPa) à une température voisine de 40°C. Le résidu pâteux est trituré en présence de 50 cm³ d'acétate d'éthyle. Le solide formé est filtré pour donner un second jet de disulfure de di-[2-(1-imidazolyl)éthyle]. Les deux jets de disulfure de di-[2-(1-imidazolyl)éthyle] sont réunis et séchés sous vide (10 kPa) à une température voisine de 20°C pour conduire à 14,2 g de disulfure de di-[2-(1-imidazolyl)éthyle].

Le 2-(1-imidazolyl)-éthanethiol peut être préparé de la manière suivante :
Une solution de 28,34 g de chlorhydrate de 2-(1-imidazolyl)éthylisothiourée et de 18.56 g d'hydroxyde de sodium dans 300 cm³ d'eau distillée est chauffée à reflux durant 150 minutes. Après avoir ramené le mélange à une température voisine de 20°C, ce dernier est acidifié par ajout d'acide chlorhydrique concentré (20 cm³) puis amené à pH = 7 par ajout d'une solution saturée de bicarbonate de sodium. Le mélange est extrait par 600 cm³ au total d'acétate d'éthyle. Les phases organiques réunies sont lavées par 100 cm³ au total d'eau distillée, filtrées, séchées sur sulfate de magnésium puis concentrées à sec sous pression réduite (2,7 kPa) à une température voisine de 40°C pour conduire à 15,0 g de 2-(1-imidazolyl)-éthanethiol sous la forme d'une huile jaune.

Le chlorhydrate de 2-(1-imidazolyl)éthylisothiourée peut être préparé de la manière suivante :
A une solution de 30 g de 2-hydroxy-(1-imidazolyl)-éthane dans 300 cm³ de dichlorométhane on ajoute goutte à goutte en 30 minutes 44,2 cm³ de chlorure de thionyle puis on porte le mélange à une température voisine du reflux durant 16 heures. Le mélange est évaporé à sec sous pression réduite (2,7 kPa) à une température voisine de 40°C. Le résidu pâteux est traité par 100 cm³ de dichlorométhane puis concentré sous pression réduite (2,7 kPa) à une température voisine de 40°C. Cette étape est répétée deux fois. Une suspension de 34,9 g de chlorhydrate de 2-chloro-(1-imidazolyl)-éthane brut ainsi obtenu et 15,93 g de thiourée dans 125 cm³ de diméthylformamide est chauffée à une température voisine de 110°C durant 90 minutes. Le mélange est refroidi à une température voisine de 20°C. Le solide jaune formé est ensuite filtré, rincé avec 100 cm³ au total d'éther de diéthyle et séché sous vide (10 kPa) à une température voisine de 40°C pour conduire à 28,34 g de chlorhydrate de 2-(1-imidazolyl)éthylisothiourée sous la forme d'un solide fondant à une température voisine de 206°C.

Le 2-hydroxy-(1-imidazolyl)-éthane peut être préparé de la manière suivante :
A une suspension de 30 g d'hydrure de sodium (à 50 % dans l'huile minérale) dans 250 cm³ de diméthylformamide on ajoute en 30 minutes une solution de 68 g d'imidazole de 250 cm³ de diméthylformamide. Le mélange est agité 90 minutes à une température voisine de 20°C, puis une solution de 50,5 g de 2-chloroéthanol dans 50 cm³ de diméthylformamide est ajoutée en une heure. Le mélange est agité 12 heures à une température voisine de 20°C puis filtré. Le filtrat est traité par 100 cm³ d'eau distillée puis concentré sous pression réduite (2,7 kPa) à une température voisine de 55°C. Le résidu pâteux est repris par 150 cm³ d'éther de pétrole, la phase liquide est décantée, et le résidu est trituré durant une heure avec 100 cm³ d'isopropanol. Le précipité formé est filtré, et le filtrat est concentré sous pression réduite (2,7 kPa) à une température voisine de 40°C. L'huile résiduelle (113,1 g) est distillée sous pression réduite (5 kPa) pour conduire à 105,7 g de 2-hydroxy-(1-imidazolyl)-éthane sous la forme d'une huile jaune distillant à une température de 180-183°C sous 5 kPa.

### Exemple 4

La [(R)-2-(N-méthyl-N-*iso*-propylamino)éthylthio-Sar]³ cyclosporine A est préparée selon la méthode suivante :
A 100 cm³ d'ammoniac maintenus à une température voisine de -33°C, on ajoute 100 mg de sodium métallique puis 100 mg de nitrate ferrique. Dès que la coloration bleue du milieu a disparu, 1,1 g de sodium métallique sont ajoutés en 15 minutes. Le mélange est agité à -33°C pendant 2 heures, une solution de 3,6 g de cyclosporine A dans 60 cm³ de tétrahydrofuranne est ajoutée goutte à goutte en approximativement 20 minutes puis une solution de 3,1 g de disulfure de di-[2-(N-méthyl-N-*iso*-propylamino)éthyle] dans 15 cm³ de tétrahydrofuranne est ajoutée en 15 minutes. Le mélange réactionnel est agité à une température voisine de -33°C durant 1 heure, puis 3 g de chlorure d'ammonium solide sont ajoutés par portions. Sous agitation, on laisse évaporer l'ammoniac en faisant passer la température du mélange de -33°C à 25°C en 12 heures. Le mélange est dilué par 100 cm³ d'éther diéthylique puis filtré. Le solide est rincé avec 100 cm³ au total d'éther diéthylique. Les phases organiques réunies sont concentrées à sec sous pression réduite (2,7 kPa), à une température voisine de 40°C. Le solide obtenu (6,3 g) est purifié par chromatographie sur une colonne de silice (0,020-0,045 mm; éluant : acétate d'éthyle/méthanol 4:1 en volumes) en recueillant des fractions de 50 cm³. Les fractions contenant le produit attendu (fractions 32 à 48) sont réunies et concentrées à sec sous pression réduite (2.7 kPa) à une température voisine de 40°C pour donner 0,640 d'une laque incolore qui, traitée par 30 cm³ d'eau distillée, donne après filtration et séchage à une température voisine de 40°C 0,390 g de [(R)-2-(N-méthyl-N-*iso*-propylamino)éthylthio-Sar]³ cyclosporine A sous la forme d'un solide blanc cassé fondant vers 70°C.

Spectre de R.M.N. ¹H (400 MHz, CDCl₃, , δ en ppm) : 1,26 (d, J = 7,5 Hz, 3H : CH₃ 8β) ; 1,37 (d, J = 7,5 Hz, 3H : CH₃ 7β) ; 2,18 (s, 3H : NCH₃ du 2 N-méthyl N-*iso*-propylaminoéthylthio en 3α) ; de 2,55 à 2,75 (mt : les 4H correspondant au SCH₂CH₂N du 2 N-méthyl N-isopropyl aminoéthylthio en 3α) ; 2,71 - 2,72 - 3,12 - 3.14 - 3,27 - 3,45 et 3,51 (7 s, 3H chacun : les 7 NCH₃) ; 2,83 (mt, 1H : NCH du 2 N-méthyl N- isopropyl aminoéthylthio en 3α) ; 3,65 (d, J = 6 Hz, 1H : OH en 1β) ; 3,77 (mt, 1H : CH 1β) ; 4,54 (mt. 1H : CH 7α) ; 4,65 (t large, J = 9 Hz, 1 H : CH 5α) ; 4,84 (mt. 1H : CH 8α) ; 4,97 (dd, J = 10,5 et 6 Hz, 1H : CH α d'une leucine) ; de 5,00 à 5.10 (mt, 2H : CH a d'une leucine et CH 2α) ; 5,13 (d, J = 11 Hz, 1H : CH 11α) ; 5.24 (dd, J = 11,5 et 4 Hz, 1H : CH α d'une leucine) : 5,33 (mt, 2H : CH=CH) ; 5,50 (d. J = 6 Hz, 1H : CH 1α) ; 5,71 (dd, J = 10,5 et 4 Hz, 1H : CH α d'une leucine) ; 5,97 (s, 1H : CH 3α) ; 7,17 (d, J = 8 Hz, 1H : CONH en 8) ; 7,34 (d, J = 9 Hz, 1H : CONH en 5) ; 7,66 (d, J = 8 Hz, 1H : CONH en 7) ; 7,95 (d, J = 10 Hz, 1H : CONH en 2).

Le disulfure de di[2-(N-méthyl-N-*iso*-propylamino)éthyle peut être préparé de la manière suivante :
A une solution de 20 g de 2-(N-*iso*-propyle-N-méthylamino)-éthanethiol dans 150 cm³ d'éther de diéthyle, on ajoute 60 cm³ d'une solution aqueuse 5 N d'hydroxyde de sodium puis on fait passer un courant d'air durant 12 heures à une température voisine de 20°C. Le mélange est extrait au moyen de 150 cm³ au total d'éther de diéthyle. Les phases organiques réunies sont lavées par 100 cm³ d'eau distillée, séchées sur sulfate de magnésium puis concentrées à sec sous pression réduite (2,7 kPa) à une température voisine de 40°C. pour conduire à 11.1 g de disulfure de di-[2-(N-méthyl-N-*iso*-propylamino)éthyle] sous la forme d'une huile incolore.

Le 2-(N-*iso*-propyle-N-méthylamino)-éthanethiol est préparé selon la méthode suivante :
Une solution de 115 cm³ de N-*iso*-propyl-N-méthylamine et de 44 cm³ d'épisulfure d'éthylène dans 400 cm³ d'éther diéthylique est chauffée à une température voisine du reflux durant 36 heures. Une distillation fractionnée sous pression réduite (2,5 kPa) du mélange réactionnel conduit à 43 g de 2-(N-*iso*-propyl-N-méthylamino)-éthanethiol sous la forme d'une huile incolore bouillant vers 60°C sous 2,5 kPa.

### Exemple 5

La [(R)-2-(N-benzyl-N-méthyl)éthylthio-Sar]³ cyclosporine A est préparée selon la méthode suivante :
A 100 cm³ d'ammoniac maintenus à une température voisine de -33°C, on ajoute 100 mg de sodium métallique puis 100 mg de nitrate ferrique. Dès que la coloration bleue du mélange a disparu, 1,0 g de sodium métallique est ajouté en 30 minutes. Le mélange est agité à -33°C pendant 1 heure, puis une solution de 3,6 g de cyclosporine A dans 60 cm³ de tétrahydrofuranne est ajoutée goutte à goutte en approximativement 15 minutes. Le mélange est agité 15 minutes à une température voisine de -33°C puis une solution de 4,4 g de N,N'-dibenzyl-N,N'-diméthylcystamine dans 15 cm³ de tétrahydrofuranne sont ajoutés en 15 minutes. Le mélange réactionnel est agité à une température voisine de -33°C durant 1 heure, puis 3 g de chlorure d'ammonium solide sont ajoutés par portions. Sous agitation, on laisse évaporer l'ammoniac en faisant passer la température du milieu de -33°C à 25°C en 12 heures. Le mélange est filtré puis rincé avec 300 cm³ au total d'éther diéthylique. Les phases organiques réunies sont concentrées à sec sous pression réduite (2,7 kPa), à une température voisine de 40°C. Le solide obtenu est trituré avec 250 cm³ au total de pentane, puis filtré. Le solide résiduel (6.2 g) est purifié par chromatographie sur une colonne de silice (0,020-0,045 mm; éluant : acétate d'éthyle pur pour les fractions de 1 à 4, acétate d'éthyle/méthanol 9:1 en volume pour les fractions de 5 à 80 et acétate d'éthyle/méthanol 8:2 en volume pour les fractions 81 à 100) et en recueillant des fractions de 100 cm³. Les fractions contenant le produit attendu (fractions 8 à 25) sont réunies et concentrées à sec sous pression réduite (2,7 kPa) à une température voisine de 40°C pour donner 3,5 g d'une meringue jaune pâle qui est purifiée par chromatographie sur colonne de silice (0,020-0,045 mm; éluant : cyclohexane/acétate d'éthyle 1:1 en volume pour les fractions de 1 à 50, et cyclohexane/acétate d'éthyle 3:7 en volume pour les fractions de 51 à 120) en recueillant des fractions de 100 cm³. Les fractions contenant le produit attendu (fractions 71 à 90) sont réunies et concentrées à sec sous pression réduite (2,7 kPa) à une température voisine de 40°C pour donner 2,3 g d'une huile incolore qui est mise en solution dans 150 cm³ d'éther de diéthyle. La solution est traitée avec 150 cm³ au total d'une solution aqueuse d'acide chlorhydrique 1 N. Le précipité formé est filtré , puis mis en solution dans 200 cm³ d'acétate d'éthyle. La phase organique est séchée sur sulfate de magnésium puis concentrée à sec sous pression réduite (2,7 kPa) à une température voisine de 40°C. Le solide résiduel est trituré avec 50 cm³ de pentane. Le pentane est décanté, puis le solide est agité 12 heures à une température voisine de 20°C avec 50 cm³ d'éther diéthylique. Le solide est filtré puis rincé avec 75 cm³ au total d'éther de diéthyle. Le solide est séché durant 12 heures, sous pression réduite (3 kPa) à une température voisine de 45°C pour conduire à 1 g de [(R)-2-(N-benzyl-N-méthyl)éthylthio-Sar]³ cyclosporine A sous la forme d'un solide blanc fondant à une température voisine de 170°C.

Spectre de R.M.N. ¹H (400 MHz, CDCl₃, à une température de 333 K, δ en ppm) : 1,28 (d, J = 7,5 Hz, 3H : CH₃ 8β) ; 1,38 (d, J = 7,5 Hz, 3H : CH₃ 7β) ; 1,64 (d, J = 5 Hz, 3H : CH₃ 1η) ; 2,61 (s large, 3H : NCH₃ du 2 N-méthyl N-benzyl aminoéthylthio en 3α) ; de 2,90 à 3,20 (mt : les 4H correspondant au SCH₂CH₂N du 2 N-méthyl N-benzyl aminoéthylthio en 3α) ; 2,73 - 2,74 - 3,09 - 3,15 - 3.26 - 3.42 et 3,52 (7 s, 3H chacun : les 7 NCH₃) ; 3,87 (mt, 1H : CH 1β) ; de 3,90 à 4,20 (mf étalé, 2H : NCH₂Ar) ; 4,53 (mt, 1H : CH 7α) ; 4,72 (t large, J = 9 Hz, 1H : CH 5α) ; 4,88 (mt, 1H : CH 8α) ; de 4,95 à 5,15 (mt, 3H : CH α de deux leucines et CH 2α) ; 5,17 (d, J = 11 Hz, 1H : CH 11α) ; 5,22 (dd, J = 11 et 4 Hz, 1H : CH α d'une leucine) ; 5,35 (mt, 2H : CH=CH) ; 5,45 (d, J = 6 Hz, 1H : CH 1α) ; 5,74 (dd, J = 10.5 et 4 Hz, 1H : CH α d'une leucine) ; 5,82 (s, 1H : CH 3α) ; 7,14 (d, J = 8 Hz, 1H : CONH en 8) ; 7.31 (d, J = 9 Hz, 1H : CONH en 5) ; de 7,35 à 7,65 (mt, 6H : H aromatiques du benzyle et CONH en 7) ; 7,90 (d, J = 10 Hz, 1H : CONH en 2).

La N,N'-dibenzyl-N,N'-diméthylcystamine est préparée de la manière suivante :
Dans une solution de 18,5 g de 2-(N-benzyl-N-méthylamino)-éthanethiol dans 100 cm³ de méthanol, et de 40 cm³ d'une solution aqueuse 5 N d'hydroxyde de sodium, on fait passer un courant d'air durant 24 heures à une température voisine de 20°C. Le méthanol est éliminé sous pression réduite (2,7 kPa). Le résidu est repris avec 100 cm³ d'eau distillée. Le mélange est extrait avec 750 cm³ au total d'éther de diéthyle. La phase organique est séchée sur sulfate de magnésium puis concentrée sous pression réduite (2,7 kPa) à une température voisine de 40°C pour conduire à 17,6 g de N,N'-di-benzyl-N,N'-diméthylcystamine sous la forme d'une huile jaune utilisée sans autre purification.

Le 2-(N-benzyl-N-méthylamino)-éthanethiol est préparé selon la méthode suivante :
Une solution de 142 cm³ de N-benzyl-N-méthylamine et de 44 cm³ d'épisulfure éthylène dans 400 cm³ d'éther diéthylique est agitée durant 144 heures à une température voisine de 45°C. Une distillation fractionnée sous pression réduite (20 kPa) du mélange conduit à 105 g de 2-(N-benzyl-N-méthylamino)-éthanethiol sous la forme d'une huile incolore bouillant entre 124 et 127°C sous 20 kPa.

### Exemple 6

La [(R)-méthylthio-Sar]³ [4'-hydroxy-MeLeu]⁴ cyclosporine A est préparée selon la méthode suivante :
Dans un réacteur contenant 12,55 g d'amidure de sodium et maintenu à une température voisine de -33°C, on introduit 500 cm³ d'ammoniac liquide, puis une solution de 25 g de [4'-hydroxy-MeLeu]⁴ cyclosporine A dans 600 cm³ de tétrahydrofuranne est ajoutée goutte à goutte et sous agitation en approximativement 10 minutes. Le mélange est agité durant 45 minutes à une température voisine de -33°C puis 38,5 g de disulfure de diméthyle sont ajoutés en approximativement 5 minutes. Le mélange réactionnel est agité à une température voisine de -33°C durant 20 minutes, puis 21,4 g de chlorure d'ammonium solide sont ajoutés progressivement. Après 10 minutes d'agitation, on laisse évaporer l'ammoniac en faisant passer la température du mélange de -33°C à 25°C en 150 minutes. Le mélange réactionnel est filtré. Le solide est lavé avec 150 cm³ au total de tétrahydrofuranne. Les phases organiques réunies sont concentrées à sec sous pression réduite (2,7 kPa), à une température voisine de 40°C. Le résidu (30.95 g) est purifié par chromatographie sur colonne de silice (0,063-0,200 mm ; éluant : dichlorométhane/acétonitrile/isopropanol 79:15:6 en volume). Les fractions contenant le produit attendu sont concentrées à sec sous pression réduite (2,7 kPa) à une température voisine de 40°C pour donner un résidu 4,4 g. Un deuxième essai est réuni avec le premier, et on purifie une seconde fois par chromatographie sur colonne de silice (0,010 mm; éluant : acétate d'éthyle). Les fractions contenant le produit attendu sont concentrées à sec. Le solide obtenu est repris deux fois dans l'éther isopropylique puis séché sous vide à une température voisine de 40°C. On obtient 4,45 g de [(R)-méthylthio-Sar]³[4'-hydroxy-MeLeu]⁴ cyclosporine A sous la forme d'un solide blanc cassé fondant vers 160°C.

Spectre de R.M.N. ¹H : (400 MHz, CDCl₃, δ en ppm) : 1,27 (d, J = 7 Hz, 3H : CH₃ 8β) ; 1,37 (d, J = 7,5 Hz. 3H : CH3 7β) ; 1,64 (d, J = 5 Hz, 3H : CH3 en 1η) ; de 1.65 à 1.80 et 2,41 (respectivement mt et dd, J = 15 et 6,5 Hz, 1H chacun : CH2 4β) ; 2,17 (s. 3H : SCH3) ; 2,47 (mt, 1H : CH 5β) ; 2,71 - 3,13 - 3,18 - 3,27 - 3.46 et 3.52 (6 s, respectivement 6H - 3H - 3H - 3H - 3H et 3H : 7 NCH3) : 3,70 (d, J = 6,5 Hz, 1H : OH en 1β) ; 3.78 (mt, 1H : CH 1β) ; 4,56 (mt, 1H : CH 7α) ; 4,67 (t, J = 9 Hz, 1H : CH 5α) ; 4,86 (mt, 1H : CH 8α) ; 5,00 (dd, J = 9 et 6 Hz, 1H : CH α d'une leucine) ; de 5,05 à 5,15 (mt, 2H : CH 2α et CH α d'une leucine) ; 5,15 (d, J = 11 Hz, 1H : CH 11α) ; de 5,25 à 5,40 (mt, 2H : CH=CH) ; 5,45 (t, J = 6,5 Hz, 1H : CH 4α) ; 5,52 (d, J = 6 Hz, 1H : CH 1α) ; 5,72 (dd, J = 10,5 et 4 Hz, 1H : CH α d'une leucine) ; 5,75 (s, 1H : CH 3α) ; 7,16 (d, J = 8 Hz, 1H : CONH en 8) ; 7,52 (d. J = 9 Hz, 1H : CONH en 5) ; 7,65 (d, J = 7,5 Hz, 1H : CONH en 7) ; 7,94 (d, J = 9,5 Hz, 1H : CONH en 2).

### Exemple 7

La [(R)-2-(1-piperidyl)éthylthio-Sar]³ [4'-hydroxy-MeLeu]⁴ cyclosporine A est préparée selon la méthode suivante :
A 100 cm³ d'ammoniac maintenus à une température voisine de -33°C, on ajoute 100 mg de sodium métallique puis 100 mg de nitrate ferrique. Dès que la coloration bleue du mélange a disparu, 1,45 g de sodium métallique sont ajoutés en 20 minutes. Le mélange est agité à -33°C pendant 1 heure, puis une solution de 5 g de [4'-hydroxy-MeLeu]⁴ cyclosporine A dans 75 cm³ de tétrahydrofuranne est ajoutée goutte à goutte en approximativement 15 minutes, puis une solution de 5,92 g de disulfure de di-[2-(1-piperidyl)éthyle] dans 25 cm³ de dioxanne est ajoutée en 10 minutes. Le mélange réactionnel est agité à une température voisine de -33°C durant 90 minutes, puis 5 g de chlorure d'ammonium solide sont ajoutés par portions. Sous agitation, on laisse évaporer l'ammoniac en faisant passer la température du mélange de -33°C à 25°C en 2 heures. Le mélange est filtré et concentré à sec sous pression réduite (2,7 kPa) à une température voisine de 35°C. L'huile jaune résiduelle (12,3 g) est triturée avec 50 cm³ de pentane. Le solide ainsi formé est filtré. Ce solide est trituré avec un mélange de 50 cm³ de pentane et 50 cm³ d'hexane. Le solide est filtré puis trituré à nouveau avec 25 cm³ d'heptane. Le solide est mis en solution dans 250 cm³ de *t*-butylméthyléther et la solution est filtrée. Le filtrat est concentré à sec sous vide (2,7 kPa) à une température voisine de 40°C pour conduire à 5,52 g d'une meringue jaune pâle. Le solide est purifié par chromatographie sur une colonne d'alumine neutre (éluant : acétate d'éthyle/cyclohexane 4:1 en volume) en recueillant des fractions de 10 cm³. Les fractions contenant le produit attendu sont concentrées à sec sous pression réduite (2,7 kPa) à une température voisine de 40°C. Le solide obtenu (1,86 g) est agité avec 25 cm³ d'acétone, puis est filtré et séché sous vide (5 kPa) à une température voisine de 35°C pour donner 1,72 g de [(R)-2-(1-piperidyl)éthylthio-Sar]³ [4'-hydroxy-MeLeu]⁴ cyclosporine A sous la forme d'un solide jaune fondant à une température voisine de 143°C.

Spectre de R.M.N. ¹H (400 MHz, CDCl₃, δ en ppm) : 1,35 (d, J = 7,5 Hz, 3H : CH₃ 7β) ; 1,62 (d, J = 5 Hz. 3H : CH₃ 7η) ; 1,71 (dd. J = 15 et 6,5 Hz : 1H correspondant à 1H du CH₂ 4β) ; de 2.20 à 3.10 (mt : les 8H correspondant au SCH₂CH₂N et aux 2 NCH₂ de la pipéridine) ; 2,70 - 3,12 - 3,22 - 3,25 - 3,45 et 3,50 (6 s, respectivement 6H - 3H - 3H - 3H - 3H - 3H : les 7 NCH₃) ; 3,66 (d, J = 6Hz, 1H : OH en 1β) ; 3,73 (mt, 1H : CH 1β) ; 4,54 (mt, 1H : CH 7α) ; 4,62 (t large, J = 9 Hz, 1H : CH 5α) ; 4,83 (mt, 1H : CH 8α) ; 4,97 (dd, J = 8 et 6 Hz, 1H : CH α d'une leucine) ; de 5,00 à 5,10 (mt. 2H : CH α d'une leucine et CH 2α) ; 5,13 (d, J = 11 Hz, 1H : CH 11α) ; de 5,25 à 5,40 (mt, 2H : CH=CH) ; 5,42 (t, J = 6,5 Hz, 1H : CH 4α) ; 5,50 (d, J = 6 Hz, 1H : CH 1α) ; 5,71 (dd, J = 10,5 et 4 Hz, 1H : CH α d'une leucine) ; 6,23 (s, 1H : CH 3α) ; 7,14 (d. J = 8 Hz, 1H : CONH en 8) ; 7,53 (d, J = 9 Hz, 1 H : CONH en 5) ; 7.62 (mt, 1H : CONH en 7) ; 7.92 (d, J = 10 Hz, 1H : CONH en 2).

Le disulfure de di-[2-(1-pipéridyl)éthyle] peut être préparé selon la méthode suivante :
A une solution de 66 g de 2-(1-pipéridyl)-éthanethiol dans 850 cm³ de dichlorométhane refroidie à 0°C, on ajoute goutte à goutte en 10 minutes, 127,7 cm³ de triéthylamine puis une solution de 57,78 g d'iode dans 250 cm³ d'éther de diéthyle. Le mélange est agité durant 30 minutes, à une température voisine de 20°C, puis repris par 5 g de sulfite de sodium en solution dans 500 cm³ d'eau distillée. Le pH de la phase aqueuse est ajusté à 9 par ajout d'une solution aqueuse saturée en carbonate de potassium. La phase organique est décantée, et la phase aqueuse est extraite par 600 cm³ au total de dichlorométhane. Les phases organiques réunies sont séchées sur sulfate de magnésium, puis concentrées à sec sous pression réduite (2,7 kPa) à une température voisine de 40°C pour conduire à 56,5 g de disulfure de di-[2-(1-pipéridyl)éthyle] sous la forme d'une huile marron utilisée sans autre purification.

Le 2-(1-pipéridyl)-éthanethiol peut être préparé selon la méthode suivante :
Une solution de 137 g de chlorhydrate de 2-(1-piperidyl)éthylisothiourée et de 84,3 g d'hydroxyde de sodium dans 1300 cm³ d'eau distillée est chauffée à reflux durant 90 minutes. Après avoir été ramené à une température voisine de 20°C, le mélange est neutralisé (pH = 7) par ajout d'acide chlorhydrique concentré. Le mélange est extrait par 450 cm³ au total de dichlorométhane. Les phases organiques réunies sont lavées par 100 cm³ au total d'eau distillée, séchées sur sulfate de magnésium, puis concentrées à sec sous pression réduite (2,7 kPa) à une température voisine de 40 °C pour conduire à 66 g de 2-(1-pipéridyl)-éthanethiol sous la forme d'une huile utilisée sans autre purification.

Le chlorhydrate de 2-(1-pipéridyl)éthylisothiourée peut être préparé de la manière suivante :
Une suspension de 100 g de chlorhydrate de N-(2-chloroéthyl)pipéridine et de 41,5 g de thiourée dans 250 cm³ de diméthylformamide est chauffée à une température voisine de 110°C durant 2 heures. Le mélange est refroidi à une température voisine de 20°C. Le solide blanc formé est ensuite filtré, rincé avec 100 cm³ au total d'éther de diéthyle et séché sous vide (10 kPa) à une température voisine de 40°C pour conduire à 134,1 g de chlorhydrate de 2-(1-pipéridyl)éthylisothiourée sous la forme d'un solide blanc fondant à une température voisine de 240°C.

### Exemple 8

La [(R)-2-(N,N-diméthylamino)éthylthio-Sar]³ [4'-hydroxy-MeLeu]⁴ cyclosporine A est préparée selon la méthode suivante :
Dans un réacteur contenant 34,17 g d'amidure de sodium et maintenu à une température voisine de -33°C, on introduit 900 cm³ d'ammoniac liquide puis une solution de 59,6 g de [4'-hydroxy-MeLeu]⁴ cyclosporine A dans 900 cm³ de *t*-butylméthyléther est ajoutée goutte à goutte et sous agitation en approximativement 10 minutes. Le mélange est agité durant 45 minutes à une température voisine de -33°C, puis 81,47 g de disulfure de di-[2-(N,N-diméthylamino)éthyle] sont ajoutés en approximativement 10 minutes. Le mélange réactionnel est agité à une température voisine de -33°C durant 20 minutes, puis 66,82 g de chlorure d'ammonium solide sont ajoutés progressivement. Après 10 minutes d'agitation, on laisse évaporer l'ammoniac en faisant passer la température du mélange de -33°C à 25°C en 150 minutes. Le mélange réactionnel est filtré. Le solide est lavé avec 320 cm³ au total de *t*-butylméthyléther. Les phases organiques réunies sont concentrées à sec sous pression réduite (2,7 kPa), à une température voisine de 40°C. L'huile résiduelle (139,1 g) est diluée dans 100 cm³ d'hexane, puis précipitée dans 700 cm³ de ce même solvant. Le solide obtenu est filtré et lavé avec 300 cm³ au total de n-hexane. Le solide blanc résiduel est séché (66,7 g) puis mis en solution dans 500 cm³ de *t*-butylméthyléther. Après ajout de 700 cm³ d'eau distillée, le pH est ajusté à 2 par ajout d'acide méthanesulfonique (∼7,3 cm³). Le mélange est agité durant 1 heure à une température voisine de 20°C. La phase organique est décantée, et la phase aqueuse est extraite par 500 cm³ au total de *t*-butylméthyléther. Le pH de la phase aqueuse est amené à 9 par ajout d'ammoniaque à 20 % (∼15 cm³). La phase aqueuse est alors extraite par 500 cm³ au total de *t*-butylméthyléther. Les phases organiques réunies sont concentrées à sec sous pression réduite (2,7 kPa) à une température voisine de 40 °C. Le solide résiduel (47,38 g) est purifié par chromatographie sur une colonne d'alumine neutre, (éluant : acétate d'éthyle/méthanol 24:1 en volume), en collectant des fractions de 100 cm³. Les fractions contenant le produit attendu (fractions 1 à 7) sont concentrées sous pression réduite (2,7 kPa) à une température voisine de 40°C pour donner 37,27 g de [(R)-2-(N,N-diméthylamino)éthylthio-Sar]³ [4'-hydroxy-MeLeu]⁴ cyclosporine A brute. Une aliquote de 3,75 g de [(R)-2-(N,N-diméthylamino)éthylthio-Sar]³ [4'-hydroxy-MeLeu]⁴ cyclosporine A brute est purifiée par chromatographie sur une colonne de silice (0,63-0,20 mm; éluant : acétonitrile-méthanol/ammoniac (28 % aq.) 85:15:1 en volume) en collectant des fractions de 50 cm³. Les fractions contenant le produit attendu sont évaporées à sec sous pression réduite (2,7 kPa) à une température voisine de 40°C pour donner 1,70 g de [(R)-2-(N,N-diméthylamino)éthylthio-Sar]³ [4'-hydroxy-MeLeu]⁴ cyclosporine A sous la forme d'un solide blanc cassé fondant vers 141°C.

Spectre de R.M.N. ¹ H (400 MHz, CDCl₃, δ en ppm) : 1,23 (d, J = 7 Hz, 3H : CH₃ 8β) ; 1,33 (d, J = 7,5 Hz, 3H : CH₃ 7β) ; 1,60 (d, J = 5 Hz, 3H : CH₃ 1η) ; 1,68 et 2,36 (2 dd, J = 15 et 6,5 Hz, 1H chacun : CH₂ 4β) ; 2,23 (s large, 6H : N(CH₃)₂ du 2-diméthyl aminoéthylthio en 3α) ; 2,40 (mt, 1H : CH 5β) ; de 2,50 à 2,85 (mt, 4H : SCH₂CH₂N du 2-diméthyl aminoéthylthio en 3α) ; 2,68 - 3,09 - 3,16 - 3,22 - 3,42 et 3,47 (6 s, respectivement 6H - 3H - 3H - 3H - 3H et 3H : 7 NCH₃) ; 3,63 (d, J = 6 Hz, 1H : OH en 1β) ; 3,72 (mt, 1H : CH 1β) ; 4,52 (mt, 1H : CH 7α) ; 4,61 (t, J = 9 Hz, 1H : CH 5α) ; 4,81 (mt, 1H : CH 8α) ; 4,95 (dd, J = 9 et 6 Hz, 1H : CH α d'une leucine) ; de 5,00 à 5,10 (mt, 2H : CH 2α et CH α d'une leucine) ; 5,10 (d, J = 11 Hz, 1H : CH 11α) ; de 5,20 à 5,35 (mt, 2H : CH=CH) ; 5,40 (t, J = 6,5 Hz, 1H : CH 4α) ; 5,47 (d, J = 6 Hz, 1H : CH 1α) ; 5,68 (dd, J = 10,5 et 4 Hz, 1H : CH α d'une leucine); 5,96 (s, 1H : CH 3α) ; 7,12 (d, J = 8 Hz, 1H : CONH en 8) ; 7,46 (d, J = 9 Hz. 1H ; CONH en 5) ; 7,60 (d, J = 7,5 Hz, 1H : CONH en 7) ; 7,92 (d, J = 9,5 Hz, 1H : CONH en 2).

La [4'-hydroxy-MeLeu]⁴ cyclosporine A peut être préparée comme décrit dans la demande de brevet européen EP 484281.

### Exemple 9

La [(R)-2-(N,N-diéthylamino)éthylthio-Sar]³ [4'-hydroxy-MeLeu]⁴ cyclosporine A est préparée selon la méthode suivante :
Dans un réacteur contenant 0,89 g d'amidure de sodium et maintenu à une température voisine de -33°C, on introduit 25 cm³ d'ammoniac puis une solution de 1,654 g de [4'-hydroxy-MeLeu]⁴ cyclosporine A dans 25 cm³ de *t*-butylméthyléther est ajoutée goutte à goutte et sous agitation en approximativement 10 minutes. Le mélange est agité durant 90 minutes à une température voisine de -33°C, puis 2,87 g de disulfure de di-[2-(N,N-diéthylamino)éthyle] sont ajoutés en approximativement 5 minutes. Le mélange réactionnel est agité à une température voisine de -33°C durant 20 minutes, puis 1,74 g de chlorure d'ammonium solide sont ajoutés progressivement. Après 10 minutes d'agitation, on laisse évaporer l'ammoniac en faisant passer la température du milieu de -33 à 25°C en 150 minutes. Le mélange réactionnel est filtré. Le solide est lavé avec 8,8 cm³ au total de *t*-butylméthyléther. Les phases organiques réunies sont concentrées à sec sous pression réduite (2.7 kPa), à une température voisine de 40°C. L'huile résiduelle est agitée durant 15 minutes avec un mélange de 5 cm³ de *t*-butylméthyléther et 80 cm³ d'heptane. Le solide obtenu est filtré puis rincé avec 15 cm³ au total de n-heptane. Le solide résiduel est mis en solution dans 25 cm³ de *t*-butylméthyléther puis 35 cm³ d'eau distillée sont ajoutés à la solution obtenue et le pH de la phase aqueuse est ajusté à 2 par ajout d'acide méthanesulfonique. La phase organique est décantée, la phase aqueuse est extraite par 30 cm³ au total de *t*-butylméthyléther. Les phases aqueuses réunies sont amenées à pH = 9 par ajout d'ammoniaque à 20 %. La phase aqueuse est alors extraite par 25 cm³ au total de *t*-butylméthyléther. Les phases organiques réunies sont lavées par 10 ml d'eau déminéralisée puis concentrées sous pression réduite (2,7 kPa) à une température voisine de 40°C. L'huile marron résiduelle (1,5 g) est purifiée par chromatographie sur une colonne d'alumine neutre éluée par un mélange d'acétate d'éthyle et d'éthanol 9:1 (en volumes). Les fractions contenant le produit attendu sont concentrées sous pression réduite (2,7 kPa) à une température voisine de 40°C pour donner 0,8 g d'un solide. Une aliquote de ce solide (0,077 g) est purifiée par chromatographie sur couche mince de silice préparative (éluant : acétonitrile-méthanol-ammoniac à 28 %, 260:40:3 en volume). La silice contenant le produit attendu est prélevée et agitée avec 5 cm³ de dichlorométhane. Après filtration et évaporation sous pression réduite (2,7 kPa) à une température voisine de 40°C de la phase organique, on obtient 39 mg de [(R)-2-(N,N-diéthylamino)éthylthio-Sar]³ [4'-hydroxy-MeLeu]⁴-cyclosporine A sous la forme d'un solide amorphe blanc cassé fondant vers 140°C.

Spectre de R.M.N. ¹H (400 MHz, CDCl₃, δ en ppm) : 1,34 (d, J = 7,5 Hz, 3H : CH₃ 7β) ; 1,60 (d, J = 5 Hz, 3H : CH₃ 1η) ; 1,68 et 2,36 (2 dd, J = 15 et 6,5 Hz, 1H chacun : CH₂ 4β) ; de 2,45 à 2,85 (mt : les 4H correspondant au SCH₂CH₂N du 2-diéthyl aminoéthylthio en 3α) ; 2,51 (mt : les 4H correspondant au 2 NCH₂ du 2-diéthyl aminoéthylthio en 3α) ; 2,70 - 3,11 - 3,18 - 3,24 - 3,44 et 3,49 (6 s, respectivement 6H - 3H - 3H - 3H - 3H et 3H : 7 NCH₃) ; 3,63 (d, J = 6 Hz, 1H : OH en 1β) ; 3,74 (mt, 1H : CH 1β) ; 4,52 (mt, 1H : CH 7α); 4,61 (t, J = 9 Hz, 1H : CH 5α) ; 4,81 (mt, 1H : CH 8α) ; 4,97 (dd, J = 9 et 7 Hz, 1H : CH α d'une leucine) ; de 5,00 à 5,10 (mt, 2H : CH 2α et CH α d'une leucine) ; 5,12 (d, J = 11 Hz, 1H : CH 11α) ; de 5,25 à 5,40 (mt, 2H : CH=CH) ; 5,40 (t, J = 6,5 Hz, 1H : CH 4α) ; 5,47 (d, J = 6 Hz, 1H : CH 1α) ; 5,68 (dd, J = 10,5 et 4 Hz, 1H : CH α d'une leucine) ; 5,96 (s, 1H : CH 3α) ; 7,13 (d, J = 8 Hz, 1H : CONH en 8) ; 7,47 (d, J = 9 Hz, 1H : CONH en 5) ; 7,61 (d, J = 7,5 Hz, 1H : CONH en 7) ; 7,89 (d, J = 9,5 Hz, 1H : CONH en 2).

Le disulfure de di-[2-(N,N-diéthylamino)éthyle] peut être préparé selon Bretschneider et al., Montatsh. Chem., 81, 385-396 (1950).

### Exemple 10

La [(R)-2-(N-méthyl-N-*t*-butylamino)éthylthio-Sar]³ [4'-hydroxy-MeLeu]⁴ cyclosporine A est préparée selon la méthode suivante :
A 720 cm³ d'ammoniac maintenus à une température voisine de -33°C, on ajoute 100 mg de sodium métallique puis 100 mg de nitrate ferrique. Dès que la coloration bleue du mélange a disparu, 6,50 g de sodium métallique sont ajoutés en 30 minutes. Le mélange est agité à -33°C pendant 90 minutes, et une solution de 24 g de [4'-hydroxy-MeLeu]⁴ cyclosporine A dans 720 cm³ de *t*-butylméthyléther est ajoutée goutte à goutte en approximativement 30 minutes, puis une solution de 3,5 g de disulfure de di-[2-(N-méthyl-N-*t*-butylamino)éthyle] dans 120 cm³ de *t*-butylméthyléther est ajoutée en 15 minutes. Le mélange réactionnel est agité à une température voisine de -33°C durant 30 minutes, puis 24 g de chlorure d'ammonium solide sont ajoutés par portions. Sous agitation, on laisse évaporer l'ammoniac en faisant passer la température du mélange de -33°C à 25°C en 12 heures. Le mélange réactionnel est filtré. Le solide est lavé avec 1800 cm³ au total de diéthyléther. Les phases organiques réunies sont concentrées sous pression réduite (2,7 kPa), à une température voisine de 40°C. L'huile résiduelle (50,3 g) est triturée durant 2 heures avec 1200 cm³ de pentane. Le solide obtenu est filtré, lavé avec 1800 cm³ d'éther de diéthyle. Le solide blanc résiduel (34,9 g) est purifié par chromatographie sur une colonne de silice (0,020-0,045 mm; éluant : dichlorométhane/éthanol 19:1 en volume). Les fractions contenant le produit attendu sont concentrées à sec sous pression réduite (2,7 kPa) à une température voisine de 40°C pour donner 8,9 g d'un solide. Ce solide, trituré 12 heures avec du pentane, donne après filtration et séchage à une température voisine de 40°C, 6,1 g de [(R)-2-(N-méthyl-N-*t*-butylamino) éthylthio-Sar]³ [4'-hydroxyMeLeu]⁴-cyclosporine A sous la forme d'un solide blanc cassé fondant vers 126-136°C.

Spectre de R.M.N. ¹H (400 MHz, CDCl₃, δ en ppm) : 1,02 (s : les 9H correspondant au C(CH₃)₃) ; 1,24 (mt : les 3H correspondant au CH₃ 8β) ; 1,33 (d, J = 7,5 Hz, 3H : CH₃ 7β) ; 1,61 (d, J = 5 Hz, 3H : CH₃ 1η) ; 1,69 et 2,36 (2 dd, J = 15 et 6,5 Hz, 1H chacun : CH₂ 4β) ; 2,18 (s large, 3H : NCH₃ du 2 N-*t*-butyl N-méthylamino-éthylthio en 3α) ; de 2,50 à 2,85 (mt : les 4H correspondant au SCH₂CH₂N du 2 N-*t*-butyl-N-méthyl aminoéthylthio en 3α) ; 2,70 - 3,11 - 3,14 - 3,24 - 3.43 et 3,48 (6 s, respectivement 6H - 3H - 3H - 3H - 3H et 3H : les 7 NCH₃) ; 3,67 (mt, 1H : OH en 1β) ; 3,72 (mt, 1H : CH 1β) ; 4,52 (mt, 1H : CH 7α) ; 4,62 (t large, J = 9 Hz, 1H : CH 5α) ; 4,82 (mt, 1H : CH 8α) ; de 4,90 à 5,10 (mt, 3H : CH α de deux leucines et CH 2α) ; 5,11 (d, J = 11 Hz, 1H: CH 11α) ; de 5,20 à 5,40 (mt, 2H : CH=CH) ; 5,41 (t, J = 6,5 Hz, 1H : CH 4α) ; 5,48 (d, J = 6 Hz, 1H : CH 1α) ; 5,68 (dd, J = 10,5 et 4 Hz, 1H : CH α d'une leucine) ; 5,87 (s, 1H : CH 3α) ; 7,14 (d. J = 8 Hz, 1H : CONH en 8); 7,48 (d, J = 9 Hz, 1H : CONH en 5); 7,64 (d, J = 8 Hz, 1H : CONH en 7) ; 7,94 (d, J = 10 Hz, 1H : CONH en 2).

Le disulfure de di-[2-(N-méthyl-N-*t*-butylamino)éthyle] peut être préparé de la manière suivante :
A une solution de 28.7 g de 2-(N-*t*-butyl-N-méthylamino)-éthanethiol dans 190 cm³ de méthanol, on ajoute 0,1 cm³ d'une solution aqueuse 1 N d'hydroxyde de sodium, puis on fait passer un courant d'air durant 60 heures à une température voisine de 20°C. Le méthanol est éliminé sous pression réduite (2,7 kPa). L'huile résiduelle est mise en solution dans 400 cm³ d'éther de diéthyle. La phase organique est séchée sur sulfate de magnésium, filtrée, puis concentrée sous pression réduite (2,7 kPa) à une température voisine de 40°C.pour conduire à 26,6 g de disulfure de di-[2-(N-méthyl-N-*t*-butylamino)éthyle] sous la forme d'une huile jaune.

Le 2-(N-*t*-butyl-N-méthylamino)-éthanethiol peut être préparé selon la méthode suivante :
Une solution de 125 cm³ de N-*t*-butyl-N-méthylamine et de 50 g d'épisulfure d'éthylène dans 750 cm³ de *t*-butylméthyléther est agitée durant 48 heures à une température voisine du reflux. Le mélange est concentré sous pression réduite (10 kPa) à une température voisine de 35°C. Une distillation fractionnée sous pression réduite (5,8 kPa) du mélange réactionnel conduit à 28,7 g de 2-(N-*t*-butyl-N-méthylamino)-éthanethiol sous la forme d'une huile incolore bouillant entre 84 et 86°C sous 5,8 kPa.

### Exemple 11

En opérant de manière analogue à la méthode décrite dans l'exemple 1, on prépare les produits suivants :
[(R)-2-aminoéthylthio-Sar]³-cyclosporine A ;
[(R)-2-(N-méthylamino)éthylthio-Sar]³-cyclosporine A ;
[(R)-2-(N-éthylamino)éthylthio-Sar]³-cyclosporine A ;
[(R)-2-(N-*i*.propylamino)éthylthio-Sar]³-cyclosporine A ;
[(R)-2-(N-*t*.butylamino)éthylthio-Sar]³-cyclosporine A ;
[(R)-2-(N-phénylamino)éthylthio-Sar]³-cyclosporine A ;
[(R)-2-(N-benzylamino)éthylthio-Sar]³-cyclosporine A ;
[(R)-2-(N-méthyl-N-éthylamino)éthylthio-Sar]³-cyclosporine A ;
[(R)-2-(N-méthyl-N-allylamino)éthylthio-Sar]³-cyclosporine A ;
[(R)-2-(N-méthyl-N-phénylamino)éthylthio-Sar]³-cyclosporine A ;
[(R)-2-(N,N-di-*i*.propylamino)éthylthio-Sar]³-cyclosporine A ;
[(R)-2-(N,N-diallylamino)éthylthio-Sar]³-cyclosporine A ;
[(R)-3-aminopropylthio-Sar)³-cyclosporine A ;
[(R)-3-(N-méthylamino)propylthio-Sar]³-cyclosporine A ;
[(R)-3-(N-éthylamino)propylthio-Sar]³-cyclosporine A ;
[(R)-3-(N-*i*.propylamino)propylthio-Sar]³-cyclosporine A ;
[(R)-3-(N-*t*.butylamino)propylthio-Sar]³-cyclosporine A ;
[(R)-3-(N-phénylamino)propylthio-Sar]³-cyclosporine A ;
[(R)-3-(N-benzylamino)propylthio-Sar]³-cyclosporine A ;
[(R)-3-(N-méthyl-N-éthylamino)propylthio-Sar]³-cyclosporine A ;
[(R)-3-(N-méthyl-N-*i*.propyllamino)propylthio-Sar]³-cyclosporine A ;
[(R)-3-(N-méthyl-N-*t*.butylamino)propylthio-Sar]³-cyclosporine A ;
[(R)-3-(N-méthyl-N-allylamino)propylthio-Sar]³-cyclosporine A ;
[(R)-3-(N-méthyl-N-phénylamino)propylthio-Sar]³-cyclosporine A ;
[(R)-3-(N-méthyl-N-benzylamino)propylthio-Sar]³-cyclosporine A ;
[(R)-3-(N,N-diéthylamino)propylthio-Sar]³-cyclosporine A ;
[(R)-3-(N,N-di-*i*.propylamino)propylthio-Sar]³-cyclosporine A ;
[(R)-3-(N,N-diallylamino)propylthio-Sar]³-cyclosporine A ;
[(R)-3-(1-pipéridyl)propylthio-Sar]³-cyclosporine A ;
[(R)-4-aminobutylthio-Sar]³-cyclosporine A ;
[(R)-4-(N-méthylamino)butylthio-Sar]³-cyclosporine A ;
[(R)-4-(N-éthylamino)butylthio-Sar]³-cyclosporine A ;
[(R)-4-(N-*i*.propylamino)butylthio-Sar]³-cyclosporine A ;
[(R)-4-(N-*t*.butylamino)butylthio-Sar]³-cyclosporine A ;
[(R)-4-(N-phénylamino)butylthio-Sar]³-cyclosporine A ;
[(R)-4-(N-benzylamino)butylthio-Sar]³-cyclosporine A ;
[(R)-4-(N-méthyl-N-éthylamino)butylthio-Sar]³-cyclosporine A ;
[(R)-4-(N-méthyl-N-*i*.propylamino)butylthio-Sar]³-cyclosporine A ;
[(R)-4-(N-méthyl-N-*t*.butylamino)butylthio-Sar]³-cyclosporine A ;
[(R)-4-(N-méthyl-N-allylamino)butylthio-Sar]³-cyclosporine A ;
[(R)-4-(N-méthyl-N-phénylamino)butylthio-Sar]³-cyclosporine A ;
[(R)-4-(N-méthyl-N-benzylamino)butylthio-Sar]³-cyclosporine A ;
[(R)-4-(N,N-diméthylamino)butylthio-Sar]³-cyclosporine A ;
[(R)-4-(N,N-diéthylamino)butylthio-Sar]³-cyclosporine A ;
[(R)-4-(N,N-di-*i*.propylamino)butylthio-Sar]³-cyclosporine A ;
[(R)-4-(N,N-diallylamino)butylthio-Sar]³-cyclosporine A ;
[(R)-4-(1-pipéridyl)butylthio-Sar]³-cyclosporine A ;
[(R)-2-amino-2-méthylpropylthio-Sar]³-cyclosporine A ;
[(R)-2-(N,N-diméthylamino)-2-méthylpropylthio-Sar]³-cyclosporine A ;
[(R)-2-(N,N-diéthylamino)-2-méthylpropylthio-Sar]³-cyclosporine A ;
[(R)-2-(1-pipéridyl)-2-méthylpropylthio-Sar]³-cyclosporine A ;
[(R)-3-amino-3-méthylbutylthio-Sar]³-cyclosporine A ;
[(R)-3-(N,N-diméthylamino)-3-méthylbutylthio-Sar]³-cyclosporine A ;
[(R)-3-(N,N-diéthylamino)-3-méthylbutylthio-Sar]³-cyclosporine A ;
[(R)-3-(1-pipéridyl)-3-méthylbutylthio-Sar]³-cyclosporine A ;
[(R)-2-(1-morpholino)éthylthio-Sar]³-cyclosporine A ;
[(R)-2-(1-azétidino)éthylthio-Sar]³-cyclosporine A ;
{(R)-2-[1-(4-méthylpipérazino)]éthylthio-Sar}³-cyclosporine A ;
{(R)-2-[1-(4-phénylpipérazino)]éthylthio-Sar}³-cyclosporine A ;
{(R)-2-[1-(4-benzylpipérazino)]éthylthio-Sar}³-cyclosporine A ;
{(R)-2-[1-(4-méthyl-1,2,3,6-tétrahydropyridyl)]éthylthio-Sar}³-cyclosporine A ;
{(R)-2-[1-(4-phényl-1,2,3,6-tétrahydropyridyl)]éthylthio-Sar}³-cyclosporine A ;
[(R)-3-( 1-morpholino)propylthio-Sar]³-cyclosporine A ;
[(R)-3-(1-azétidino)propylthio-Sar]³-cyclosporine A ;
{(R)-3-[1-(4-méthylpipérazino)]propylthio-Sar}³-cyclosporine A ;
{(R)-3-[1-(4-phénylpipérazino)]propylthio-Sar}³-cyclosporine A ;
{(R)-3-[1-(4-benzylpipérazino)]propyithio-Sar}³-cyclosporine A ;
{(R)-3-[1-(4-méthyl-1,2,3,6-tétrahydropyridyl)]propylthio-Sar}³-cyclosporine A ;
{(R)-3-[1-(4-méthyl-1,2,3,6-tétrahydropyridyl)]propylthio-Sar}³-cyclosporine A.
[(R)-2-aminoéthylthio-Sar]³-[4'-hydroxy-MeLeu]⁴-cyclosporine A ;
[(R)-2-(N-méthylamino)éthylthio-Sar]³-[4'-hydroxy-MeLeu]⁴-cyclosporine A ;
[(R)-2-(N-éthylamino)éthylthio-Sar]³-[4'-hydroxy-MeLeu]⁴-cyclosporine A ;
[(R)-2-(N-*iso*-propylamino)éthylthio-Sar]³-[4'-hydroxy-MeLeu]⁴-cyclosporine A ;
[(R)-2-(N-*t*-butylammo)éthylthio-Sar]³-[4'-hydroxy-MeLeu]⁴-cyclosporine A ;
[(R)-2-(N-phénylamino)éthylthio-Sar]³-[4'-hydroxy-MeLeu]⁴-cyclosporine A ;
[(R)-2-(N-benzylamino)éthylthio-Sar]³-[4'-hydroxy-MeLeu]⁴-cyclosporine A ;
[(R)-2-(N-méthyl-N-éthylamino)éthylthio-Sar]³-[4'-hydroxy-MeLeu]⁴-cyclosporine A;
[(R)-2-(N-méthy[-N-iso-propylamino)éthylthio-Sar]³-[4'-hydroxy-MeLeu]⁴-cyclosporine A ;
[(R)-2-(N-méthyl-N-tert-butylamino)éthylthio-Sar]³-[4'-hydroxy-MeLeu]⁴-cyclosporine A ;
[(R)-2-(N-méthyl-N-allylamino)éthylthio-Sar]³-[4'-hydroxy-MeLeu]⁴-cyclosporine A;
[(R)-2-(N-méthyl-N-phénylamino)éthylthio-Sar]³-(4'-hydroxy-MeLeu]⁴-cyclosporine A ;
[(R)-2-(N-méthyl-N-benzylamino)éthylthio-Sar]³-[4'-hydroxy-MeLeu]⁴-cyclosporine A ;
[(R)-2-(N,N-diéthylamino)éthylthio-Sar]³-[4'-hydroxy-MeLeu]⁴-cyclosporine A ;
[(R)-2-(N,N-di-iso-propylamino)éthylthio-Sar]³-[4'-hydroxy-MeLeu]⁴-cyclosporine A ;
[(R)-2-(N,N-diallylamino)éthylthio-Sar]³-[4'-hydroxy-MeLeu]⁴-cyclosporine A ;
[(R)-2-(1-pipéridyl)éthylthio-Sar]³-[4'-hydroxy-MeLeu]⁴-cyclosporine A ;
[(R)-3-aminopropylthio-Sar]³-[4'-hydroxy-MeLeu]⁴-cyclosporine A ;
[(R)-3-(N-méthylamino)propylthio-Sar]³-[4'-hydroxy-MeLeu]⁴-cyclosporine A ;
[(R)-3-(N-éthylamino)propylthio-Sar]³-[4'-hydroxy-MeLeu]⁴-cyclosporine A ;
[(R)-3-(N-iso-propylamino)propylthio-Sar]³-[4'-hydroxy-MeLeu]⁴-cyclosporine A ;
[(R)-3-(N-tert-butylamino)propylthio-Sar]³-[4'-hydroxy-MeLeu]⁴-cyclosporine A ;
[(R)-3-(N-phénylamino)propylthio-Sar]³-[4'-hydroxy-MeLeu]⁴-cyclosporine A ;
[(R)-3-(N-benzylamino)propylthio-Sar]³-[4'-hydroxy-MeLeu]⁴-cyclosporine A ;
[(R)-3-(N-méthyl-N-éthylamino)propylthio-Sar]³-[4'-hydroxy-MeLeu]⁴-cyclosporine A ;
[(R)-3-(N-méthyl-N-*i*-propylamino)propylthio-Sar]³-[4'-hydroxy-MeLeu]⁴-cyclosporine A ;
[(R)-3-(N-méthyl-N-*t*-butylamino)propylthio-Sar]³-[4'-hydroxy-MeLeu]⁴-cyclosporine A ;
[(R)-3-(N-méthyl-N-allylamino)propylthio-Sar]³-[4'-hydroxy-MeLeu]⁴-cyclosporine A ;
[(R)-3-(N-méthyl-N-phénylamino)propylthio-Sar]³-[4'-hydroxy-MeLeu]⁴-cyclosporine A ;
[(R)-3-(N-méthyl-N-benzylamino)propylthio-Sar]³-[4'-hydroxy-MeLeu]⁴-cyclosporine A ;
[(R)-2-(N,N-diméthylamino)éthylthio-Sar]³-[4'-hydroxy-MeLeu]⁴-cyclosporine A ;
[(R)-3-(N,N-diéthylamino)propylthio-Sar]³-[4'-hydroxy-MeLeu]⁴-cyclosporine A ;
[(R)-3-(N,N-di-*i*-propylamino)propylthio-Sar]³-[4'-hydroxy-MeLeu]⁴-cyclosporine A ;
[(R)-3-(N,N-diallyalamino)propylthio-Sar]³-[4'-hydroxy-MeLeu]⁴-cyclosporine A ;
[(R)-3-(1-pipéridyl)propylthio-Sar]³-[4'-hydroxy-MeLeu]⁴-cyclosporine A ;
[(R)-4-aminobutylthio-Sar]³-[4'-hydroxy-MeLeu]⁴-cyclosporine A ;
[(R)-4-(N-méthylamino)butylthio-Sar]³-[4'-hydroxy-MeLeu]⁴-cyclosporine A ;
[(R)-4-(N-éthylamino)butylthio-Sar]³-[4'-hydroxy-MeLeu]⁴-cyclosporine A :
[(R)-4-(N-*i*-propylamino)butylthio-Sar]³-[4'-hydroxy-MeLeu]⁴-cyclosporine A :
[(R)-4-(N-*t*-butylamino)butylthio-Sar]³-[4'-hydroxy-MeLeu]⁴-cyclosporine A ;
[(R)-4-(N-phénylamino)butylthio-Sar]³-[4'-hydroxy-MeLeu]⁴-cyclosporine A ;
[(R)-4-(N-benzylamino)butylthio-Sar]³-[4'-hydroxy-MeLeu]⁴-cyclosporine A ;
[(R)-4-(N-méthyl-N-éthylamino)butylthio-Sar]³-[4'-hydroxy-MeLeu]⁴-cyclosporine A ;
[(R)-4-(N-méthyl-N-*i*-propylamino)butylthio-Sar]³-[4'-hydroxy-MeLeu]⁴-cyclosporine A ;
[(R)-4-(N-méthyl-N-*t*-butylamino)butylthio-Sar]³-[4'-hydroxy-MeLeu]⁴-cyclosporine A ;
[(R)-4-(N-méthyl-N-allylamino)butylthio-Sar]³-[4'-hydroxy-MeLeu]⁴-cyclosporine A ;
[(R)-4-(N-méthyl-N-phénylamino)butylthio-Sar]³-[4'-hydroxy-MeLeu]⁴-cyclosporine A ;
[(R)-4-(N-méthyl-N-benzylamino)butylthio-Sar]³-[4'-hydroxy-MeLeu]⁴-cyclosporine A ;
[(R)-4-(N,N-diméthylamino)butylthio-Sar]³-[4'-hydroxy-MeLeu]⁴-cyclosporine A ;
[(R)-4-(N,N-diéthylamino)butylthio-Sar]³-[4'-hydroxy-MeLeu]⁴-cyclosporine A ;
[(R)-4-(N,N-di-*i*-propylamino)butylthio-Sar]³-[4'-hydroxy-MeLeu]⁴-cyclosporine A ;
[(R)-4-(N,N-diallyalamino)butylthio-Sar]³-[4'-hydroxy-MeLeu]⁴-cyclosporine A ;
[(R)-4-(1-pipéridyl)butylthio-Sar]³-[4'-hydroxy-MeLeu]⁴-cyclosporine A ;
[(R)-2-amino-2-méthylpropylthio-Sar]³-[4'-hydroxy-MeLeu]⁴-cyclosporine A ;
[(R)-2-(N,N-diméthylamino)-2-méthylpropylthio-Sar]³-[4'-hydroxy-MeLeu]⁴-cyclosporine A ;
[(R)-2-(N,N-diéthylamino)-2-méthylpropylthio-Sar]³-[4'-hydroxy-MeLeu]⁴-cyclosporine A ;
[(R)-2-(1-pipéridyl)-2-méthylpropylthio-Sar]³-[4'-hydroxy-MeLeu]⁴-cyclosporine A ;
[(R)- 3-amino-3-méthylbutylthio-Sar]³-[4'-hydroxy-MeLeu]⁴-cyclosporine A ;
[(R)-3-(N,N-diméthylamino)-3-méthylbutylthio-Sar]³-[4'-hydroxy-MeLeu]⁴-cyclosporine A ;
[(R)-3-(N,N-diéthylamino)-3-méthylbutylthio-Sar]³-[4'-hydroxy-MeLeu]⁴-cyclosporine A ;
[(R)-3-(1-pipéridyl)-3-méthylbutylthio-Sar]³-[4'-hydroxy-MeLeu]⁴-cyclosporine A ;
[(R)-2-(1-morpholino)éthylthio-Sar]³-[4'-hydroxy-MeLeu]⁴-cyclosporine A ;
[(R)-2-(1-azétidino)éthylthio-Sar]³-[4'-hydroxy-MeLeu]⁴-cyclosporine A ;
{(R)-2-[1-(4-méthylpipérazino)]éthylthio-Sar}³-[4'-hydroxy-MeLeu]⁴-cyclosporine A ;
{(R)-2-[1-(4-phénylpipérazino)]éthylthio-Sar}³-[4'-hydroxy-MeLeu]⁴-cyclosporine A ;
{(R)-2-[1-(4-benzylpipérazino)]éthylthio-Sar}³-[4'-hydroxy-MeLeu]⁴-cyclosporine A ;
{(R)-2-[1-(4-méthyl-1,2,3,6-tétrahydropyridyl)]éthylthio-Sar}³-[4'-hydroxy-MeLeu]⁴-cyclosporine A ;
{(R)-2-[1-(4-phényl-1,2,3,6-tétrahydropyridyl)]éthylthio-Sar}³-[4'-hydroxy-MeLeu]⁴-cyclosporine A ;
[(R)-3-( 1-morpholino)propylthio-Sar]³-[4'-hydroxy-MeLeu]⁴-cyclosporine A :
[(R)-3-(1-azétidino)propylthio-Sar]³-[4'-hydroxy-MeLeu]⁴-cyclosporine A ;
{(R)-3-[ 1-(4-méthylpipérazino)]propylthio-Sar}³-[4'-hydroxy-MeLeu]⁴-cyclosporine A ;
{(R)-3-[1-(4-phénylpipérazino)]propylthio-Sar}³-[4'-hydroxy-MeLeu]⁴-cyclosporine A ;
{(R)-3-[1-(4-benzylpipérazino)]propylthio-Sar}³-[4'-hydroxy-MeLeu]⁴-cyclosporine A ;
{(R)-3-[1-(4-méthyl-1,2,3,6-tétrahydropyridyl)]propylthio-Sar}³-[4'-hydroxy-MeLeu]⁴-cyclosporine A ;
{(R)-3-[1-(4-méthyl-1,2,3,6-tétrahydropyridyl)]propylthio-Sar}³-[4'-hydroxy-MeLeu]⁴-cyclosporine A.

## Revendications

1. Procédé de préparation d'un polyanion utile pour la préparation de dérivés de cyclosporine modifiés en position -3 **caractérisé en ce que** l'on traite une cyclosporine par un amidure alcalin dans un mélange binaire comprenant de l'ammoniac liquide ou une amine aliphatique de bas poids moléculaire et un cosolvant, éventuellement en présence de diméthylpropylènurée (DMPU).

2. Procédé selon la revendication 1 pour la préparation d'un polyanion utile pour la préparation de dérivés de cyclosporine **caractérisé en ce que** ledit polyanion a pour formule : 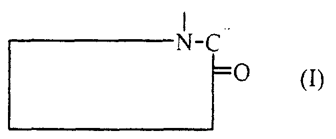 dans laquelle 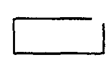 représente une cyclosporine sur laquelle un ou des groupements hydroxy et/ou un ou des atomes d'azote non-méthylés en position α et/ou tout autre groupement acide déprotonable sont éventuellement déprotonés, ou sont sous forme protégée.

3. Procédé selon l'une des revendications 1 ou 2 **caractérisé en ce que** le polyanion a pour formule générale : 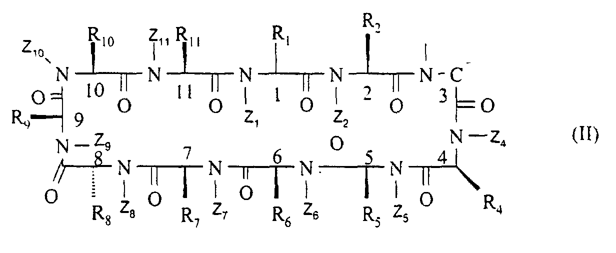 pour laquelle :
i) Les substituants R₁ à R₁₁ et Z₁ à Z₁₁ sont définis comme pour la cyclosporine A, ou bien
ii) Les substituants R₁ à R₁₁ et Z₁ à Z₁₁ sont définis comme pour la cyclosporine A, à l'exception de R₄ et Z₄ qui sont définis de façon à avoir en position -4 l'acide aminé 4'-(hydroxy)méthylleucine (MeLeu), ou bien
iii) Les substituants R₂, R₅ à R₁₁ et Z₂, Z₅ à Z₁₁ sont définis comme pour la cyclosporine A, et
• Z₁ est un groupement méthyle, et R₁ a pour formule : 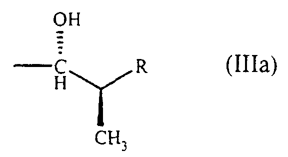
- R étant un groupement de formule -CH₂-CH=CH-CH₂-R' pour lequel R' représente un radical alkylthio, aminoalkylthio, alkylaminoalkylthio, dialkylaminoalkylthio, pynmidinylthio, thiazolylthio, N-alkylimidazolylthio, hydroxyalkylphénylthio, hydroxyalkylphényloxy, nitrophénylamino, ou 2-oxopyrimidin-1-yle, ou
- R étant un groupement de formule -CH₂-S-Alk pour lequel Alk représente un groupement alkyle, et
• Z₄ et R₄ sont des radicaux tels que l'on a en position -4 un acide aminé MeLeu ou 4'-(hydroxy)MeLeu, ou bien,
iv) Z₁ et R₁ sont des groupements tels que l'on a en position -1 une homothréonine substituée de formule générale :
Rᵢ-CH₂CH(CH₃)-CH(OH)-CH(NHCH₃)-COOH (IIIb)
dans laquelle Rᵢ représente le *n*-propyle ou le propényle et la double liaison présente de préférence une configuration trans, et
• R₂ et Z₂ sont des radicaux tels que l'on a en position -2 l'acide alpha-aminobutyrique (αAbu), la valine (Val), la norvaline (Nva), ou la thréonine (Thr), et
• R₄ et Z₄ sont des radicaux tels que l'on a en position -4 la N-méthyl-gamma-hydroxy-leucine ou la N-méthyl-gamma-acétyloxyleucine, et
• R₅ et Z₅ sont des radicaux tels que l'on a en position -5 la valine, et
• R₆, Z₆, R₉, Z₉, R₁₀ et Z₁₀ sont des radicaux tels que l'on a en position -6. -9, et -10 la N-méthylleucine, et
• Z₇ et R₇ sont des radicaux tels que l'on a en position -7 l'alanine (Ala), et
• Z₈ et R₈ sont des radicaux tels que l'on a en position -8 la (D)-alanine ou la (D)-sérine, et
• Z₁₁ et R₁₁ sont des radicaux tels que l'on a en position - 11 la N-méthyl-valine, ou bien,
v) Z ₁ et R₁ sont des groupements tels que l'on a en position -1 un substituant méthyl-(4R)-4-[(E)-2-butènyl-4-méthyl-L-thréonine (MeBmt), ou un substituant ayant pour formule générale : 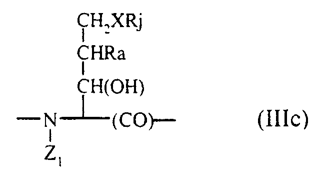 Rⱼ représentant un atome d'hydrogène, ou un groupement alkyle inférieur, un alcényle inférieur, un halogénoalkyle inférieur, un aryle, un alkyloxy inférieur, un alkoxy C₁₋₆ alkyle, un hydroxyméthyle, un alkylthio inférieur, un alkylthioC₁₋₆alkyle, un mercaptoC₁₋₆alkyle, ou un hétéroaryle, les groupements aryle et hétéroaryle pouvant être substitués avec un ou plusieurs groupements fonctionnels alkyle C₁₋₆, alcanoyle C₁₋₆, halogénoalkyle C₁₋₆, halogène, cyano, hydroxyalkyle C₁₋₃, alkyloxy C₁₋₆, alkyl-S(O)ₙ C₁₋₆ avec n = 0, 1, ou 2, NR_{b}COR_{c} avec R_{b} et R_{c} représentant indépendamment H ou un alkyle C₁₋₆, -NO₂, -NR_{b}R_{c}, -OR_{b}, -CONR_{b}R_{c}, -COR_{b}, -NR_{b}CONR_{b}R_{c}. NR_{b}COR_{c}, -OCOR_{b}, -SCOR_{b}, ou -OCH₂O-, et Rₐ étant un alkyle inférieur, Z₁ étant un alkyle inférieur, un phénylalkyle inférieur, ou un aryle, et X représentant S, SO, SO₂, O, ou bien NR_{b}, et
• Z₂ et R₂ sont des substituants tels que l'on a en position -2 l'acide aminé L-2-aminobutyryle, norvalyle, L-thréonyle, ou bien le même acide aminé qu'en position -1, et
• Z₄ et R₄ sont des substituants tels que l'on a en position -4 l'acide aminé N-méthyl-L-leucyle, et
• Z₅ et R₅ sont des substituants tels que l'on a en position -5 l'acide aminé L-valyle, ou norvalyle, et
• Z₆ et R₆ sont des substituants tels que l'on a en position -6 l'acide aminé N-méthyl-L-leucyle, et
• Z₇ et R₇ sont des substituants tels que l'on a en position -7 l'acide aminé L-alanyle, L-2-aminobutyryle, ou L-phénylalanyle, et
• Z₈ et R₈ sont des substituants tels que l'on a en position -8 l'acide aminé D-alanyle ou L-alanyle, et
• Z₉ et R₉ sont des substituants tels que l'on a en position -9 l'acide aminé N-méthyl-L-leucyle ou N-méthyl-L-valyle, et
• Z₁₀ et R₁₀ sont des substituants tels que l'on a en position -10 l'acide aminé N-méthyl-L-leucyle ou L-leucyle, et
• Z₁₁ et R₁₁ sont des substituants tels que l'on a en position -11 l'acide aminé N-méthyl-L-valyle, L-valyle, ou L-2-aminobutyryle, ou bien
vi) Les substituants R₄ à R₁₁, et Z₄ à Z₁₁ sont définis comme pour la cyclosporine A, et : Z₁ et R₁ sont des substituants tels que l'on a en position -1 l'acide aminé MeBmt ou dihydro-MeBmt, et
• Z₂ et R₂ sont des substituants tels que l'on a en position -2 l'acide aminé αAbu, Thr, Val, ou Nva, ou bien
vii) Les substituants R₇ à R₁₁, et Z₇ à Z₁₁ sont définis comme pour la cyclosporine A, et :
• Z₁ et R₁ sont des substituants tels que l'on a en position -1 l'acide aminé MeBmt, dihydro-MeBmt, ou 8'-hydroxy-MeBmt, et
• Z₂ et R₂ sont des substituants tels que l'on a en position -2 l'acide aminé αAbu, Val, Thr, Nva, ou méthyl-O-thréonine (MeOThr), et
• Z₄ et R₄ sont des substituants tels que l'on a en position -4 l'acide aminé MeLeu, γ-hydroxy-MeLeu, Melle, MeVal, MeThr, MeAla, MeaIle, ou MeaThr, et
• Z₅ et R₅ sont des substituants tels que l'on a en position -5 l'acide aminé Val, Leu, MeVal, ou MeLeu, et
• Z₆ et R₆ sont des substituants tels que l'on a en position -6 l'acide aminé MeLeu, γ-hydroxy-MeLeu, ou MeAla,
à condition que lorsque R₄ et Z₄ signifient MeLeu, R₅ et Z₅ signifient alors MeVal ou MeLeu ou bien R₁ et Z₁ signifient 8'-hydroxy-MeBmt, ou bien
viii) Les substituants R₁ à R₁₁ et Z₁ à Z₁₁ définissent une cyclosporine pour laquelle le carbone en 3' du résidu en position -1 ou le carbone en β du résidu en position-2 est substitué par O-acyle ou oxo, et notamment
• Z₁ et R₁ sont des substituants tels que l'on a en position -1 un résidu de formule générale 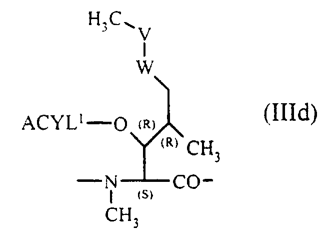 pour laquelle -v-w- est CH₂-CH₂ ou CH=CH trans et ACYL¹ représente un groupe acyle, et
• Z₂ et R₂ sont des substituants tels que l'on a en position -2 un acide aminé αAbu, Val, Thr, Nva, ou le résidu d'un α-acide aminé β-O-acylé, et
• Z₅ et R₅ sont des substituants tels que l'on a en position -5 un acide aminé Val, ou Nva lorsque l'on a simultanément un acide aminé Nva en position -2, et
• Z₈ et R₈ sont des substituants tels que l'on a en position -8 un acide aminé (D)-Ala, un résidu d'un α-acide aminé β-O-acylé ou β-hydroxylé ayant la configuration (D), et
• les substituants en position -4, -6, -7, et -9 à -11 sont définis comme pour la cyclosporine A,
un ou des groupements hydroxy et/ou un ou des atomes d'azote non-méthylés en position α et/ou tout autre groupement acide déprotonable présents dans ladite formule générale (II) étant éventuellement déprotonés, ou étant sous forme protégée.

4. Procédé selon l'une des revendications 1 à 3 **caractérisé en ce que** l'amidure alcalin est l'amidure de sodium, l'amidure de potassium, le sel de lithium de l'hexaméthyldisilazane, ou le sel de sodium de l'hexaméthyldisilazane.

5. Procédé selon l'une des revendications 1 à 3 **caractérisé en ce que** la cyclosporine est traitée par 10 à 20 moles d'amidure alcalin par mole de cyclosporine.

6. Procédé selon l'une des revendications 1 à 3 **caractérisé en ce que** le mélange binaire contient 40 % à 60 % en volume d'ammoniac liquide ou d'amine aliphatique de bas poids moléculaire.

7. Procédé selon l'une des revendications 1 à 3 **caractérisé en ce que** l'amine aliphatique de bas poids moléculaire est la méthylamine.

8. Procédé selon l'une des revendications 1 à 3 **caractérisé en ce que** le cosolvant est choisi parmi les éthers aliphatiques ou cycliques, les hydrocarbures aromatiques, ou la pyridine.

9. Procédé selon la revendication 8 **caractérisé en ce que** le cosolvant est choisi parmi le tétrahydrofuranne, le *t*-butylméthyléther, le diméthoxyéthane, l'anisole, le dioxanne ou le toluène.

10. Procédé selon l'une des revendications 1 à 3 **caractérisé en ce qu'**on opère à température comprise entre -38°C et la température d'ébullition de l'ammoniac liquide ou de l'amine aliphatique de bas poids moléculaire.

11. Procédé selon l'une des revendications 1 à 3 **caractérisé en ce que** le ratio (poids/poids) de cyclosporine mis en jeu par rapport au poids total de la solution est inférieur ou égal à 15%.

12. Procédé de préparation de dérivés de cyclosporine substitués en position -3 **caractérisé en ce que** l'on prépare un polyanion par traitement d'une cyclosporine par un amidure alcalin dans un mélange binaire comprenant de l'ammoniac liquide ou une amine aliphatique de bas poids moléculaire et un cosolvant, éventuellement en présence de diméthylpropylènurée, puis on additionne un agent électrophile, les radicaux hydroxy présents sur la cyclosporine et/ou les substituants de l'agent électrophile pouvant éventuellement interférer avec la réaction étant préalablement protégés, et élimine le cas échéant les radicaux protecteurs et/ou transforme éventuellement le produit obtenu en un sel lorsqu'ils existent.

## Claims

1. Process for the preparation of a polyanion which is suitable for the preparation of cyclosporin derivatives which are modified at the 3-position, **characterized in that** a cyclosporin is treated with an alkali metal amide in a two-way mixture comprising liquid ammonia or a low-molecular-weight aliphatic amine and a cosolvent, optionally in the presence of dimethylpropyleneurea (DMPU).

2. Process according to Claim 1 for the preparation of a polyanion which is suitable for the preparation of cyclosporin derivatives, **characterized in that** said polyanion has the formula 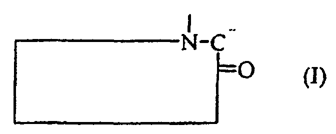 in which 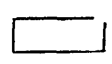 represents a cyclosporin at which one or more hydroxyl groups and/or one or more nonmethylated nitrogen atoms at the α-position and/or any other deprotonatable acidic group are optionally deprotonated or are in the protected form.

3. Process according to Claim 1 or 2, **characterized in that** the polyanion has the general formula 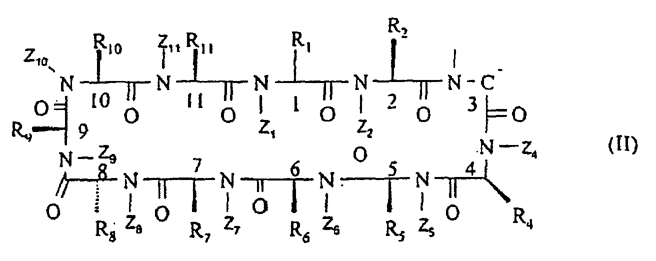 in which
i) the substituents R₁ to R₁₁ and Z₁ to Z₁₁ are defined as for cyclosporin A, or else
ii) the substituents R₁ to R₁₁ and Z₁ to Z₁₁ are defined as for cyclosporin A, with the exception of R₄ and Z₄, which are defined so as to have, at the 4-position, the amino acid 4'-(hydroxymethylleucine (MeLeu), or else
iii) the substituents R₂, R₅ to R₁₁ and Z₂, Z₅ to Z₁₁ are defined as for cyclosporin A, and
• Z₁ is a methyl group and R₁ has the formula 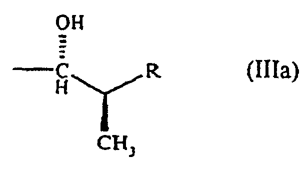
- R being a group of formula -CH₂-CH=CH-CH₂-R' in which R' represents an alkylthio, aminoalkylthio, alkylaminoalkylthio, dialkylaminoalkylthio, pyrimidinylthio, thiazolylthio, N-alkylimidazolylthio, hydroxyalkylphenylthio, hydroxyalkylphenyloxy, nitrophenylamino or 2-oxopyrimidin-1-yl radical, or
- R being a group of formula -CH₂-S-Alk in which Alk represents an aryl group, and
• Z₄ and R₄ are radicals such that there is, at the 4-position, an amino acid MeLeu or 4'-(hydroxy)-MeLeu, or else,
iv) Z₁ and R₁ are groups such that there is, at the 1-position, a substituted homothreonine of the general formula
Rᵢ-CH₂CH(CH₃)-CH (OH) -CH (NHCH₃) -COOH (IIIb)
in which Rᵢ represents n-propyl or propenyl and the double bond preferably exhibits a trans configuration and
• R₂ and Z₂ are radicals such that there is, at the 2-position, α-aminobutyric acid (αAbu), valine (Val), norvaline (Nva) or threonine (Thr), and
• R₄ and Z₄ are radicals such that there is, at the 4-position, N-methyl-gamma-hydroxyleucine or N-methyl-gamma-acetyloxyleucine, and
• R₅ and Z₅ are radicals such that there is, at the 5-position, valine, and
• R₆, Z₆, R₉, Z₉, R₁₀ and Z₁₀ are radicals such that there is, at the 6-, 9- and 10-position, N-methylleucine, and
• Z₇ and R₇ are radicals such that there is, at the 7-position, alanine (Ala), and
• Z₈ and R₈ are radicals such that there is, at the 8-position, (D)-alanine or (D)-serine, and
• Z₁₁ and R₁₁ are radicals such that there is, at the 11-position, N-methylvaline, or else,
v) Z₁ and R₁ are groups such that there is, at the 1-position, a methyl-(4R)-4-[(E)-2-butenyl-4-methyl-L-threonine (MeBmt) substituent or a substituent having the general formula 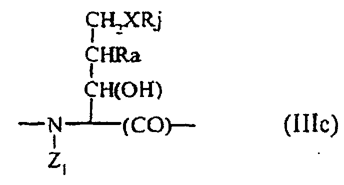 Rⱼ representing a hydrogen atom or a lower alkyl group, a lower alkenyl, a lower haloalkyl, an aryl, a lower alkyloxy, an alkoxyC₁₋₆alkyl, a hydroxymethyl, a lower alkylthio, an alkylthioC₁₋₆alkyl, a C₁₋₆ mercaptoalkyl or a heteroaryl, it being possible for the aryl and heteroaryl groups to be substituted by one or more of the following functional groups: C₁₋₆ alkyl, C₁₋₆ alkanoyl, C₁₋₆ haloalkyl, halogen, cyano, C₁₋₃ hydroxyalkyl, C₁₋₆ alkyloxy, C₁₋₆ alkyl-S (O)ₙ where n = 0, 1 or 2, NR_{b}COR_{c} with R_{b} and R_{c} independently representing H or a C₁₋₆ alkyl, -NO₂, -NR_{b}R_{c}, -OR_{b}, -CONR_{b}R_{c}, -COR_{b}, -NR_{b}CONR_{b}R_{c}, NR_{b}COR_{c}, -OCOR_{b}, -SCOR_{b} or -OCH₂O-, and Rₐ being a lower alkyl, Z₁ being a lower alkyl, a lower phenylalkyl or an aryl and X representing S, SO, SO₂, O or else NR_{b}, and
• Z₂ and R₂ are substituents such that there is, at the 2-position, the amino acid L-2-aminobutyryl, norvalyl, L-threonyl or else the same amino acid as at the 1-position, and
• Z₄ and R₄ are substituents such that there is, at the 4-position, the amino acid N-methyl-L-leucyl, and
• Z₅ and R₅ are substituents such that there is, at the 5-position, the amino acid L-valyl or norvalyl, and
• Z₆ and R₆ are substituents such that there is, at the 6-position, the amino acid N-methyl-L-leucyl, and
• Z₇ and R₇ are substituents such that there is, at the 7-position, the amino acid L-alanyl, L-2-aminobutyryl or L-phenylalanyl, and
• Z₈ and R₈ are substituents such that there is, at the 8-position, the amino acid D-alanyl or L-alanyl, and
• Z₉ and R₉ are substituents such that there is, at the 9-position, the amino acid N-methyl-L-leucyl or N-methyl-L-valyl, and
• Z₁₀ and R₁₀ are substituents such that there is, at the 10-position, the amino acid N-methyl-L-leucyl or L-leucyl, and
• Z₁₁ and R₁₁ are substituents such that there is, at the 11-position, the amino acid N-methyl-L-valyl, L-valyl or L-2-aminobutyryl, or else
vi) the substituents R₄ to R₁₁ and Z₄ to Z₁₁ are defined as for cyclosporin A, and Z₁ and R₁ are substituents such that there is, at the 1-position, the amino acid MeBmt or dihydro-MeBmt, and
• Z₂ and R₂ are substituents such that there is, at the 2-position, the amino acid αAbu, Thr, Val or Nva, or else
vii) the substituents R₇ to R₁₁ and Z₇ to Z₁₁ are defined as for cyclosporin A, and
• Z₁ and R₁ are substituents such that there is, at the 1-position, the amino acid MeBmt, dihydro-MeBmt or 8'-hydroxy-MeBmt, and
• Z₂ and R₂ are substituents such that there is, at the 2-position, the amino acid αAbu, Val, Thr, Nva or methyl-O-threonine (MeOThr), and
• Z₄ and R₄ are substituents such that there is, at the 4-position, the amino acid MeLeu, γ-hydroxy-MeLeu, MeIle, MeVal, MeThr, MeAla, MeaIle or MeaThr, and
• Z₅ and R₅ are substituents such that there is, at the 5-position, the amino acid Val, Leu, MeVal or MeLeu, and
• Z₆ and R₆ are substituents such that there is, at the 6-position, the amino acid MeLeu, γ-hydroxy-MeLeu, or MeAla,
with the proviso that, when R₄ and Z₄ mean MeLeu, R₅ and Z₅ then mean MeVal or MeLeu or else R₁ and Z₁ mean 8'-hydroxy-MeBmt, or else
viii) the substituents R₁ to R₁₁ and Z₁ to Z₁₁ define a cyclosporin in which the 3' carbon of the residue at the 1-position or the β carbon of the residue at the 2-position is substituted by O-acyl or oxo, and in particular
• Z₁ and R₁ are substituents such that there is, at the 1-position, a residue of the general formula 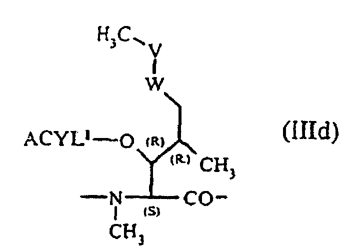 in which -v-w- is CH₂-CH₂, or trans CH=CH and ACYL¹ represents an acyl group, and
• Z₂ and R₂ are substituents such that there is, at the 2-position, an amino acid αAbu, Val, Thr or Nva or the residue of a β-O-acylated α-amino acid, and
• Z₅ and R₅ are substituents such that there is, at the 5-position, an amino acid Val, or Nva when there is simultaneously an amino acid Nva at the 2-position, and
• Z₈ and R₈ are substituents such that there is, at the 8-position, an amino acid (D)-Ala or a residue of a β-O-acylated or β-hydroxylated α-amino acid having the (D) configuration, and
• the substituents at the 4-, 6-, 7- and 9- to 11-position are defined as for cyclosporin A,
one or more hydroxyl groups and/or one or more nonmethylated nitrogen atoms at the α-position and/or any other deprotonatable acidic group present in said general formula (II) optionally being deprotonated or being in the protected form.

4. Process according to any of Claims 1 to 3, **characterized in that** the alkali metal amide is sodium amide, potassium amide, the lithium salt of hexamethyldisilazane or the sodium salt of hexamethyldisilazane.

5. Process according to any of Claims 1 to 3, **characterized in that** the cyclosporin is treated with 10 to 20 mol of alkali metal amide per mol of cyclosporin.

6. Process according to any of Claims 1 to 3, **characterized in that** the two-way mixture comprises 40% to 60% by volume of liquid ammonia or low-molecular-weight aliphatic amine.

7. Process according to any of Claims 1 to 3, **characterized in that** the low-molecular-weight aliphatic amine is methylamine.

8. Process according to any of Claims 1 to 3, **characterized in that** the cosolvent is chosen from aliphatic or cyclic ethers, aromatic hydrocarbons or pyridine.

9. Process according to Claim 8, **characterized in that** the cosolvent is chosen from tetrahydrofuran, t-butyl methyl ether, dimethoxyethane, anisole, dioxane or toluene.

10. Process according to any of Claims 1 to 3, **characterized in that** the process is carried out at a temperature of between -38°C and the boiling point of the liquid ammonia or the low-molecular-weight aliphatic amine.

11. Process according to any of Claims 1 to 3, **characterized in that** the (weight/weight) ratio of cyclosporin employed relative to the total weight of the solution is 15% or less.

12. Process for the preparation of cyclosporin derivatives substituted at the 3-position, **characterized in that** a polyanion is prepared by treating a cyclosporin with an alkali metal amide in a two-way mixture comprising liquid ammonia or a low-molecular-weight aliphatic amine and a cosolvent, optionally in the presence of dimethylpropyleneurea, then an electrophilic agent is added, the hydroxyl radicals present on the cyclosporine and/or the substituents of the electrophilic agent which may possibly interfere with the reaction being protected beforehand, and then, if appropriate, the protective radicals are removed and/or the product obtained is optionally converted to a salt, when they exist.

## Patentansprüche

1. Verfahren zur Herstellung eines Polyanions, das sich für die Herstellung von in 3-Stellung modifizierten Cyclosporinderivaten eignet, **dadurch gekennzeichnet, daß** man ein Cyclosporin mit einem Alkalimetallamid in einer Zweiermischung, die flüssigen Ammoniak oder ein niedermolekulares aliphatisches Amin und ein Hilfslösungsmittel enthält, gegebenenfalls in Gegenwart von Dimethylpropylenharnstoff (DMPH), behandelt.

2. Verfahren nach Anspruch 1 zur Herstellung eines Polyanions, das sich für die Herstellung von Cyclosporinderivaten eignet, **dadurch gekennzeichnet, daß** das Polyanion die folgende Formel aufweist: 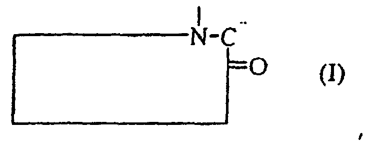 in der 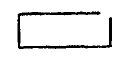 ein Cyclosporin bedeutet, an dem eine oder mehrere Hydroxygruppen und/oder ein oder mehrere unmethylierte Stickstoffatome in α-Stellung und/oder beliebige andere entprotonisierbare saure Gruppen gegebenenfalls entprotonisiert werden oder in geschützter Form vorliegen.

3. Verfahren nach Anspruch 1 oder 2, **dadurch gekennzeichnet, daß** das Polyanion der folgenden allgemeinen Formel entspricht: 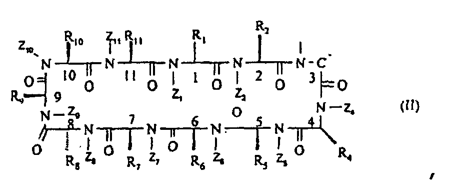 in der
i) die Substituenten R₁ bis R₁₁ und Z₁ bis Z₁₁ wie oben für Cyclosporin A definiert sind, oder
ii) die Substituenten R₁ bis R₁₁ und Z₁ bis Z₁₁ wie für Cyclosporin A definiert sind, außer daß R₄ und Z₄ so definiert sind, daß sie in 4-Stellung die Aminosäure 4'-(Hydroxy)methylleucin (MeLeu) aufweisen, oder
iii) die Substituenten R₂, R₅ bis R₁₁ und Z₂, Z₅ bis Z₁₁ wie für Cyclosporin A definiert sind, und
• Z₁ eine Methylgruppe bedeutet und R₁ die folgende Formel aufweist: 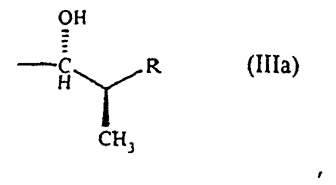 wobei
R eine Gruppe der Formel -CH₂-CH=CH-CH₂-R' bedeutet, in der R' einen Alkylthio-, Aminoalkylthio-, Alkylaminoalkylthio-, Dialkylaminoalkylthio-, Pyrimidinylthio-, Thiazolylthio-, N-Alkylimidazolylthio-, Hydroxyalkylphenylthio-, Hydroxyalkylphenyloxy-, Nitrophenylamino- oder 2-Oxopyrimidin-1-ylrest bedeutet, oder
R eine Gruppe der Formel -CH₂-S-Alk bedeutet, in der Alk eine Alkylgruppe bedeutet, und
• Z₄ und R₄ solche Reste sind, daß in 4-Stellung eine Aminosäure MeLeu oder 4'-(Hydroxy)MeLeu vorliegt, oder
iv) Z₁ und R₁ sind solche Gruppen, daß in 1-Stellung ein substituiertes Homothreonin der allgemeinen Formel
Rᵢ-CH₂CH (CH₃) -CH (OH) -CH (NHCH₃) -COOH (IIIb),
in der Rᵢ n-Propyl oder Propenyl bedeutet und die Doppelbindung vorzugsweise eine trans-Konfiguration aufweist, vorliegt und
• R₂ und Z₂ solche Reste sind, daß in 2-Stellung die Aminosäure alpha-Aminobuttersäure (αAbu), Valin, (Val), Norvalin (Nva) oder Threonin (Thr) vorliegt und
• R₄ und Z₄ solche Reste sind, daß in 4-Stellung N-Methyl-gamma-hydroxyleucin oder N-Methylgamma-acetyloxyleucin vorliegen, und
• R₅ und Z₅ solche Reste sind, daß in 5-Stellung Valin vorliegt, und
• R₆, Z₆, R₉, Z₉ R₁₀ und Z₁₀ solche Reste sind, daß in 6-, 9- und 10-Stellung N-Methylleucin vorliegt, und
• Z₇ und R₇ solche Reste sind, daß in 7-Stellung Alanin (Ala) vorliegt, und
• Z₈ und R₈ solche Reste sind, daß in 8-Stellung (D)-Alanin oder (D)-Serin vorliegen, und
• Z₁₁ und R₁₁ solche Reste sind, daß in 11-Stellung N-Methylvalin vorliegt, oder
v) Z₁ und R₁ solche Gruppen sind, daß in 1-Stellung ein Methyl-(4R)-4-[(E)-2-butenyl-4-methyl-Lthreonin (MeBmt)-Substituent oder ein Substituent, der der allgemeinen Formel 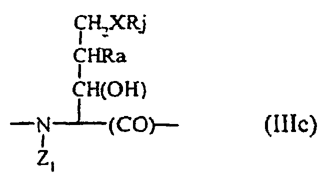 entspricht, vorliegt,
wobei Rⱼ ein Wasserstoffatom oder eine Niederalkylgruppe, Niederalkenyl, Niederhalogenalkyl, Aryl, Niederalkyloxy, Alkoxy-C₁₋₆-Alkyl, Hydroxymethyl, Niederalkylthio, Alkylthio-C₁₋₆-Alkyl, C₁₋₆-Mercaptoalkyl oder Heteroaryl bedeutet, wobei die Aryl- und Heteroarylgruppen durch eine oder mehrere funktionelle Gruppen C₁₋₆-Alkyl, C₁₋₆-Alkanoyl, C₁₋₆-Halogenalkyl, Halogen, Cyano, C₁₋₃-Hydroxyalkyl, C₁₋₆-Alkyloxy, C₁₋₆-Alkyl-S(O)ₙ, wobei n = 0, 1 oder 2, NR_{b}COR_{c}, wobei R_{b} und R_{c} unabhängig H oder C₁₋₆-Alkyl, -NO₂, -NR_{b}R_{c}, -OR_{b}, -CONR_{b}R_{c}, -COR_{b}, -NR_{b}CONR_{b}R_{c}, NR_{b}COR_{c}, -OCOR_{b}, SCOR_{b} oder -OCH₂O- bedeuten, substituiert sein können, und
Rₐ Niederalkyl bedeutet, Z₁ Niederalkyl, Niederphenylalkyl oder Aryl bedeutet und X S, SO, SO₂, O oder auch NR_{b} bedeutet, und
• Z₂- und R₂ solche Reste sind-,- daß in 2-Stellung die Aminosäure L-2-Aminobutyryl, Norvalyl, L-Threonyl oder auch die gleiche Aminosäure wie in 1-Stellung vorliegt, und
• Z₄ und R₄ solche Substituenten sind, daß in 4-Stellung die Aminosäure N-Methyl-L-leucyl vorliegt, und
• Z₅ und R₅ solche Substituenten sind, daß in 5-Stellung die Aminosäure L-Valyl oder Norvalyl vorliegt, und
• Z₆ und R₆ solche Substituenten sind, daß in 6-Stellung die Aminosäure N-Methyl-L-leucyl vorliegt, und
• Z₇ und R₇ solche Substituenten sind, daß in 7-Stellung die Aminosäure L-Alanyl, L-2-Aminobutyryl oder L-Phenylalanyl vorliegt, und
• Z₈ und R₈ solche Substituenten sind, daß in 8-Stellung die Aminosäure D-Alanyl oder L-Alanyl vorliegt, und
• Z₉ und R₉ solche Substituenten sind, daß in 9-Stellung die Aminosäure N-Methyl-L-leucyl oder N-Methyl-L-valyl vorliegt, und
• Z₁₀ und R₁₀ solche Substituenten sind, daß in 10-Stellung die Aminosäure N-Methyl-L-leucyl oder L-Leucyl vorliegt, und
• Z₁₁ und R₁₁ solche Substituenten sind, daß in 11-Stellung die Aminosäure N-Methyl-L-valyl, L-Valyl oder L-2-Aminobutyryl vorliegt, oder
vi) die Substituenten R₄ bis R₁₁ und Z₄ bis Z₁₁ wie für Cyclosporin A definiert sind und Z₁ und R₁ solche Substituenten sind, daß in 1-Stellung die Aminosäure MeBmt oder Dihydro-MeBmt vorliegt, und
• Z₂ und R₂ solche Substitution sind, daß in 2-Stellung die Aminosäure αAbu, Thr, Val oder Nva vorliegt, oder
vii) die Substituenten R₇ bis R₁₁ und Z₇ is Z₁₁ wie für Cyclosporin A definiert sind und
• Z₁ und R₁ solche Substituenten sind, daß in 1-Stellung die Aminosäure MeBmt, Dihydro-MeBmt oder 8'-Hydroxy-MeBmt vorliegt, und
• Z₂ und R₂ solche Substituenten sind, daß in 2-Stellung die Aminosäure αAbu, Val, Thr, Nva oder Methyl-O-threonin (MeOThr) vorliegt, und
• Z₄ und R₄ solche Substituenten sind, daß in 4-Stellung die Aminosäure MeLeu, γ-Hydroxy-MeLeu, Melle, MeVal, MeThr, MeAla, Mealle oder MeaThr vorliegt, und
• Z₅ und R₅ solche Substituenten sind, daß in 5-Stellung die Aminosäure Val, Leu, MeVal oder MeLeu vorliegt, und
• Z₆ und R₆ solche Substituenten sind, daß in 6-Stellung die Aminosäure MeLeu, γ-Hydroxy-MeLeu oder MeAla vorliegt,
unter der Voraussetzung, daß wenn R₄ und Z₄ MeLeu bedeuten, R₅ und Z₅ MeVal oder MeLeu bedeuten oder auch R₁ und Z₁ 8'-Hydroxy-MeBmt bedeuten, oder
viii) die Substituenten R₁ bis R₁₁ und Z₁ bis Z₁₁ ein Cyclosporin definieren, bei dem der 3'-Kohlenstoff des Rests in 1-Stellung oder der β-Kohlenstoff des Rests in 2-Stellung durch O-Acyl oder Oxo substituiert ist, und insbesondere
• Z₁ und R₁ solche Substituenten sind, daß in 1-Stellung ein Rest der allgemeinen Formel 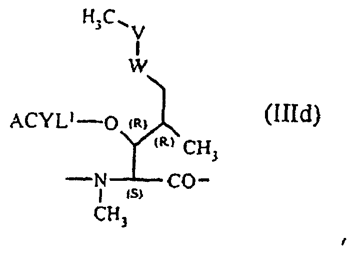 in der -v-w- CH₂-CH₂ oder trans-CH=CH bedeutet und ACYL¹ eine Acylgruppe bedeutet, entspricht, und
• Z₂ und R₂ solche Substituenten sind, daß in 2-Stellung eine Aminosäure αAbu, Val, Thr, Nva oder der Rest einer β-O-acylierten α-Aminosäure vorliegt, und
• Z₅ und R₅ solche Substituenten sind, daß in 5-Stellung eine Aminosäure Val oder, wenn gleichzeitig eine Aminosäure Nva in 2-Stellung vorhanden ist, Nva vorliegt, und
• Z₈ und R₈ solche Substituenten sind, daß in 8-Stellung eine Aminosäure (D)-Ala oder ein Rest einer β-O-acylierten oder β-hydroxylierten α-Aminosäure mit (D)-Konfiguration vorliegt, und
• die Substituenten in 4-, 6-, 7- und 9- bis 11-Stellung wie oben für Cyclosporin A definiert sind,
wobei eine oder mehrere Hydroxygruppen und/oder ein oder mehrere unmethylierte Stickstoffatome in α-Stellung und/oder beliebige andere entprotonisierbare saure Gruppen in dieser allgemeinen Formel (II) gegebenenfalls entprotonisiert sind oder in geschützter Form vorliegen.

4. Verfahren nach einem der Ansprüche 1 bis 3, **dadurch gekennzeichnet, daß** es sich bei dem Alkalimetallamid um Natriumamid, Kaliumamid, das Hexamethyldisilazan-Lithiumsalz oder das Hexamethyldisilazan-Natriumsalz handelt.

5. Verfahren nach einem der Ansprüche 1 bis 3, **dadurch gekennzeichnet, daß** Cyclosporin mit 10 bis 20 mol Alkalimetallamid pro mol Cyclosporin behandelt wird.

6. Verfahren nach einem der Ansprüche 1 bis 3, **dadurch gekennzeichnet, daß** die Zweiermischung 40 Volumenprozent bis 60 Volumenprozent flüssigen Ammoniak oder niedermolekulares aliphatisches Amin enthält.

7. Verfahren nach einem der Ansprüche 1 bis 3, **dadurch gekennzeichnet, daß** es sich bei dem niedermolekularen aliphatischen Amin um Methylamin handelt.

8. Verfahren nach einem der Ansprüche 1 bis 3, **dadurch gekennzeichnet, daß** das Hilfslösungsmittel aus der Gruppe der aliphatischen oder cyclischen Ether, der aromatischen Kohlenwasserstoffe oder Pyridin stammt.

9. Verfahren nach Anspruch 8, **dadurch gekennzeichnet, daß** das Hilfslösungsmittel aus der Gruppe Tetrahydrofuran, t-Butylmethylether, Dimethoxyethan, Anisol, Dioxan oder Toluol stammt.

10. Verfahren nach einem der Ansprüche 1 bis 3, **dadurch gekennzeichnet, daß** man bei einer Temperatur zwischen -38°C und der Siedetemperatur des flüssigen Ammoniak oder des niedermolekularen aliphatischen Amins arbeitet.

11. Verfahren nach einem der Ansprüche 1 bis 3, **dadurch gekennzeichnet, daß** das (Gewichts/Gewichts)-Verhältnis zwischen verwendetem Cyclosporin und Gesamtgewicht der Lösung 15% oder weniger beträgt.

12. Verfahren zur Herstellung von in 3-Stellung substituierten Cyclosporinderivaten, **dadurch gekennzeichnet, daß** ein Polyanion dadurch hergestellt wird, daß man ein Cyclosporin mit einem Alkalimetallamid in einer Zweiermischung, die flüssigen Ammoniak oder ein niedermolekulares aliphatisches Amin und ein Hilfslösungsmittel enthält, gegebenenfalls in Gegenwart von Dimethylpropylenharnstoff behandelt, dann ein Elektrophil zugibt, wobei die an dem Cyclosporin vorhandenen Hydroxyreste und/oder die Substituenten des Elektrophils, die gegebenenfalls die Reatkion stören können, zuvor geschützt werden, und dann falls erforderlich die Schutzreste entfernt werden und/oder gegebenenfalls das erhaltene Produkt in ein Salz umgewandelt wird, wo solche existieren.
